# EUROPEAN PATENT APPLICATION

(11) **EP 4 837 580 A1**
(43) Date of publication of application: **11.11.2026**
(21) Application number: 25174337.3
(22) Date of filing: 05.05.2025
(51) Int. Cl.: A61K 31/575, A61B 5/00, A61K 31/192, A61K 31/194, A61K 31/195, A61K 31/216, A61K 31/4406, A61K 31/4418, A61P 1/16

(54) **USE OF NORUCHOLIC ACID IN THE TREATMENT OF PRIMARY SCLEROSING CHOLANGITIS**

(71) Applicant: Dr. Falk Pharma GmbH, 79108 Freiburg (DE)
(72) Inventor: The designation of the inventor has not yet been filed
(74) Representative: Arth, Hans-Lothar

(57) **Abstract**

The present invention relates to norucholic acid for use in the treatment of primary sclerosing cholangitis in a patient which is a human, wherein the patient has a blood level of serum alkaline phosphatase of less than or equal 4.0 times upper limit of normal (ULN), and the patient has a liver stiffness of less than or equal 15.0 kPa as measured by an elastometry technique such as vibration-controlled transient elastography, and wherein the norucholic acid is administered orally to the patient at a daily dose between 500 mg and 2000 mg.

## Description

### Field of the invention

The present invention relates to norucholic acid for use in the treatment of primary sclerosing cholangitis in a patient which is a human, wherein the patient has a blood level of serum alkaline phosphatase of less than or equal 4.0 times upper limit of normal (ULN), and the patient has a liver stiffness of less than or equal 15.0 kPa as measured by an elastometry technique such as vibration-controlled transient elastography, and wherein the norucholic acid is administered orally to the patient at a daily dose between 500 mg and 2000 mg.

### Background of the invention

Primary sclerosing cholangitis (PSC) is a rare, chronic, cholestatic liver disease characterized by progressive inflammation and fibrosis of the intrahepatic and extrahepatic bile ducts. Although the exact pathogenesis remains poorly understood, PSC is widely considered an autoimmune-mediated disorder involving a complex interplay of genetic, immunologic, biliary, and gut-derived factors.

Histologically, PSC is often associated with concentric periductal fibrosis-commonly referred to as "onion-skin" scarring-reflecting chronic bile duct injury. Emerging evidence also implicates defects in protective mechanisms against bile acid toxicity as central contributors to disease development.

PSC exhibits a strong association with inflammatory bowel disease (IBD), particularly ulcerative colitis (UC), which is diagnosed in up to 80% of PSC patients in certain populations, especially in Europe and North America. The intestinal phenotype in PSC-associated IBD (PSC-IBD) frequently differs from classic UC or Crohn's disease in both location and inflammatory activity. Several mechanisms have been proposed to underlie the gut-liver axis dysfunction observed in PSC, including altered bile acid signaling, intestinal microbiota dysbiosis, aberrant lymphocyte homing, and increased intestinal permeability ("leaky gut").

Notably, PSC confers an elevated risk for hepatobiliary and colorectal malignancies, underscoring the urgent need for effective therapeutic interventions. Currently, diagnosis of PSC relies on cholangiographic imaging to visualize characteristic bile duct changes.

Despite extensive research, no pharmacological treatment has yet been approved that alters the natural course of PSC. Management remains largely symptomatic.

Ursodeoxycholic acid (UDCA), administered at doses of 15-20 mg/kg/day, may improve biochemical markers such as liver enzymes; however, current clinical evidence does not support its use as a disease-modifying therapy. Moreover, high-dose UDCA (28-30 mg/kg/day) has been associated with increased rates of serious adverse events and a higher incidence of clinical endpoints including death, liver transplantation, progression to cirrhosis, and development of cholangiocarcinoma, despite transient improvements in laboratory values. A comprehensive meta-analysis (Triantos et al. Aliment Pharmacol Ther 2011; 34: 901-910) concluded that neither standard nor high-dose UDCA provides a significant benefit in terms of improving survival, alleviating symptoms (e.g., pruritus, fatigue), reducing cancer risk, or preventing histological disease progression in PSC. As such, no current treatment has demonstrated efficacy in improving hard clinical outcomes.

This persistent lack of effective therapeutic options highlights the urgent and unmet medical need for novel, targeted interventions capable of modifying disease progression and improving long-term outcomes in PSC patients.

WO201272689A1 relates to a scalable industrial process for producing 24-nor-ursodeoxycholic acid ("norucholic acid", "Nor-UDCA" or "NCA") or Bis-nor-UDCA with high purity and pharmaceutical suitability. The process involves purification and optional recrystallization of the potassium salt of NCA, followed by precipitation of the free acid, yielding a novel, physically pure, and thermodynamically stable crystalline form of NCA, designated "Form A".

Halibasic et al. (2017) Dig Dis 35:288-292 summarizes the recent progress of Nor-UDCA as a novel therapeutic approach in cholestatic and metabolic liver disorders with specific focus on primary sclerosing cholangitis (PSC).

WO 2019/63790 A1 discloses the use of norucholic acid ("Nor-UDCA" or "NCA") for reducing liver fat in patients with hepatic steatosis, including those without inflammation or hepatitis. The treatment is particularly effective at doses above 500 mg/day and also benefits patients with mild steatosis and normal ALT levels. Additionally, NCA was found to lower serum levels of cytokeratin 18 fragments, markers of cell death (apoptosis or necrosis), suggesting a protective effect on the liver.

Thus, it is the objective of the present invention to enable the identification of patients who are likely to respond positively to treatment with norucholic acid ("Nor-UDCA", herein referred to as "NCA"). More specifically, the invention aims to provide reliable means to distinguish between responders and non-responders to NCA therapy in the treatment of primary sclerosing cholangitis (PSC) or related cholestatic liver conditions.

The objective of the present invention is solved by the teaching of the independent claims. Further advantageous features, aspects and details of the invention are evident from the dependent claims, the description, the figures, and the examples of the present application.

### Description of the invention

Surprisingly, the inventors have found that human patients suffering from primary sclerosing cholangitis (PSC) can be efficiently treated with norucholic acid (NCA), when
a) the patient has a blood level of serum alkaline phosphatase (ALP) of less than or equal 4.0 times upper limit of normal (ULN), and
b) the patient has a liver stiffness of less than or equal 15.0 kPa as measured by an elastometry technique such as Vibration-Controlled Transient Elastography (VCTE), and
wherein the norucholic acid is administered orally to the patient at a daily dose between 500 mg and 2000 mg.

A critical goal of this invention is to reduce the clinical and psychological burden of ineffective therapy on non-responders. Patients who do not derive benefit from NCA may be unnecessarily subjected to prolonged treatment regimens that offer no therapeutic gain, while still incurring the risk of adverse effects. Notably, NCA treatment may be associated with undesirable side effects such as pruritus, which can significantly impair quality of life. In non-responders, side effects including nasopharyngitis (primarily viral, bacterial, or chronic forms) and headache (notably tension-type, migraine, medication-induced, secondary, and post-infectious headaches) occur without offsetting clinical benefit, thereby exacerbating the risk-benefit imbalance of the therapy.

By enabling the early identification of likely responders, the invention facilitates personalized treatment strategies, reduces unnecessary healthcare costs, minimizes patient exposure to potential harm, and enhances overall therapeutic efficacy.

The inventors thus identified that patients having a blood level of serum alkaline phosphatase of less than or equal 4.0 times upper limit of normal (ULN), and which have a liver stiffness of less than or equal 15.0 kPa as measured by an elastometry technique such as vibration-controlled transient elastography (VCTE), respond particularly positively to the oral administration of NCA.

The inventors have surprisingly found that liver stiffness may serve as a reliable non-invasive biomarker for assessing the degree of hepatic fibrosis in PSC patients. A reduction or stabilization in liver stiffness values over time following NCA treatment may indicate a therapeutic response, whereas progressive increases may be indicative of non-responsiveness or fibrosis advancement.

Thus, within the context of the present invention, a "responder" is defined as a subject who exhibits a statistically or clinically significant decrease in the level of one or more biomarkers (e.g., ALP, GGT, ALT, AST, ELF, liver stiffness) from baseline to a defined follow-up time point (e.g., week 12, week 96), following oral administration of the NCA treatment.

In contrast, a "non-responder" is defined as a subject who exhibits a statistically or clinically insignificant change or no change in the level of one or more biomarkers (e.g., ALP, GGT, ALT, AST, ELF, liver stiffness) from baseline to a defined follow-up time point (e.g., week 12, week 96), following oral administration of the NCA treatment in comparison to a placebo group. More precisely, a responder is defined herein as a subject having
(i) a baseline blood level of serum alkaline phosphatase (ALP) of less than or equal 4.0 times upper limit of normal (ULN), which reduces below 1.5 times ULN after 12 weeks of NCA administration and
(ii) a baseline liver stiffness of less than or equal 15.0 kPa as measured by an elastometry technique such as Vibration-Controlled Transient Elastography (VCTE), which is maintained or reduced during 96 weeks of NCA administration.

As used herein, a "non-responder" is defined as a subject who fails to exhibit a statistically or clinically significant improvement in one or more disease-associated biomarkers (e.g., ALP, GGT, ALT, AST, ELF, liver stiffness) following oral administration of the NCA treatment, as compared to a placebo group over a defined treatment period.

More precisely, a non-responder is defined as a subject meeting one or both of the following conditions:
(i) A subject having a baseline blood level of serum alkaline phosphatase (ALP) greater than 4.0 times the upper limit of normal (ULN),
   and/or
(ii) A subject having a baseline liver stiffness greater than 15.0 kPa, as measured by an elastometry technique such as Vibration-Controlled Transient Elastography (VCTE), Acoustic Radiation Force Impulse (ARFI) imaging, Shear Wave Elastography (SWE), Magnetic Resonance Elastography (MRE), Supersonic Shear Imaging Elastometry, Transient Elastography (TE), or Magnetic Resonance Imaging (MRI)-based stiffness quantification.

As used herein, "remission, cure, or resolution rate" refers to the percentage of patients that are cured or obtain remission or complete resolution of a condition in response to a given treatment. Quantitatively, a patient responds to a treatment positively when (i) the patient's blood level of serum alkaline phosphatase decreases and is maintained below 1.5 times ULN after at least 8 weeks NCA treatment, and (ii) no worsening of fibrosis stage according to Ludwig's Classification from baseline to week 96. Remission, cure, or resolution of primary sclerosing cholangitis refers to reduced or no sign of symptoms selected from the group comprising fever, abdominal pain, anemia, itching, and liver dysfunction.

As used herein, "response rate" refers to the percentage of patients that responds positively to a given NCA treatment as defined above.

As used herein, the term "treating" refers to (i) completely or partially inhibiting a disease, disorder or condition, for example, arresting its development; (ii) completely or partially relieving a disease, disorder or condition, for example, causing regression of the disease, disorder and/or condition; or (iii) completely or partially preventing a disease, disorder or condition from occurring in a patient that may be predisposed to the disease, disorder and/or condition, but has not yet been diagnosed as having it. Similarly, "treatment" refers to both therapeutic treatment and prophylactic or preventative measures.

As used herein, "therapeutically effective amount" or "pharmaceutically active dose" refers to an amount of a composition which is effective in treating the named disease, disorder or condition.

As used herein, the term "Vibration-controlled Transient Elastography" or "VCTE" refers to a commonly applied method for determination of the liver stiffness measured in Pascal (Pa). This method is described in detail in the following reference: Elliot B. Tapper, Laurent Castera, Nezam H. Afdhal, FibroScan (Vibration-Controlled Transient Elastography): Where Does It Stand in the United States Practice, Clinical Gastroenterology and Hepatology, Volume 13, Issue 1, 2015, Pages 27-36.

As used herein, the term "upper limit of normal (ULN)" refers to the upper boundary of the reference range for a given clinical laboratory parameter, as established by the testing laboratory for a healthy population. For serum alkaline phosphatase (ALP), this is typically in the range of 120 to 140 IU/L in adults, though exact values may vary depending on the laboratory and assay used. A value expressed as a multiple of ULN (e.g., "1.5× ULN" or "1.5 times ULN") means 1.5 times the laboratory's upper reference limit.

As used herein, the term "elevated levels" refers to blood concentrations of liver-associated biomarkers that exceed the upper limit of normal (ULN) as defined by standard clinical laboratory reference ranges. Such biomarkers include, but are not limited to: Alkaline phosphatase (ALP), Gamma-glutamyltransferase (GGT), Alanine aminotransferase (ALT), and Aspartate aminotransferase (AST). In one embodiment the biomarkers are ALP, ALT and AST. Example reference ULN values for adults may be approximately: ALP: 120 IU/L, GGT: 40 IU/L (men), 30 IU/L (women), ALT: 45 IU/L (men), 34 IU/L (women), and AST: 35 IU/L.

In one embodiment "elevated levels" in the context of the present invention are given in absolute elevations, where the measured value exceeds a defined IU/L threshold. For example: ALP > 180 IU/L, GGT > 60 IU/L, ALT > 68 IU/L, and/or AST > 53 IU/L.

In one embodiment "elevated levels" in the context of the present invention are given in relative elevations, expressed as multiples of the ULN; e. g. mild elevation: >1.1 to ≤1.5 × ULN; moderate elevation: >1.5 to ≤3.0 × ULN; marked elevation: >3.0 to ≤5.0 × ULN; and severe elevation: >5.0 × ULN.

In a preferred embodiment, a biomarker is considered elevated when it is equal to or greater than 1.5 times the ULN (i.e. ≥ 1.5 times the ULN).

For the purposes of the present invention, a level may be referred to as "elevated" if it is ≥1.5 × ULN, unless otherwise specified.

As used herein, the term "baseline" refers to the initial reference point at which one or more physiological, biochemical, or clinical parameters are measured in a patient prior to the initiation of a treatment with NCA. Baseline values are used as a comparator to assess the effectiveness, progression, or response to the treatment over a defined time period. Unless otherwise specified, baseline measurements are taken immediately before the first administration of the therapeutic compound or commencement of the therapeutic regimen described in the present invention.

As used herein, the expression "from baseline to week ..." refers to the time interval beginning at the baseline, the initial time point at which a patient's condition, biomarker level, or clinical status is first assessed, prior to the initiation of treatment and extending through to the end of the specified week of the treatment or observation period. This interval represents the duration over which changes in clinical, biochemical, or other evaluative parameters are measured in order to assess the effect or efficacy of the therapeutic intervention described in the present invention.

The Ludwig staging system is a semiquantitative histopathological classification method used to assess the stage of liver fibrosis in chronic cholestatic liver diseases, including primary sclerosing cholangitis (PSC). The stages are defined as follows:
- Stage 1: Portal fibrosis without septa.
- Stage 2: Periportal fibrosis with rare portal-portal septa.
- Stage 3: Numerous septa without cirrhosis (bridging fibrosis).
- Stage 4: Cirrhosis.

"No progression of Ludwig stage" means that a patient's liver biopsy at follow-up (e.g., week 96) shows the same or a lower fibrosis stage compared to baseline, with no increase in fibrosis grade.

As used herein, anti-nuclear antibodies (ANA) are a class of autoantibodies directed against nuclear components of cells, commonly detected by indirect immunofluorescence. ANA positivity is often observed in autoimmune liver diseases and is present in a subset of patients with primary sclerosing cholangitis.

pANCA refers to a staining pattern in immunofluorescence assays representing autoantibodies that bind to the perinuclear region of neutrophils. pANCA is frequently positive in patients with ulcerative colitis and PSC, and is considered a supportive serologic marker in diagnosing immune-mediated cholangiopathies.

AMA are autoantibodies directed against mitochondrial antigens, especially the E2 subunit of the pyruvate dehydrogenase complex. AMA positivity is a hallmark of primary biliary cholangitis (PBC). In contrast, AMA is typically negative in PSC. Therefore, AMA negativity is useful in differentiating PSC from PBC in diagnostic workup.

As used herein, the terms "norucholic acid", "norursodeoxycholic acid", "nor-ursodeoxycholic acid", "NCA", and "Nor-UDCA" refer to the compound 24-nor-ursodeoxycholic acid, which has the following structural formula:

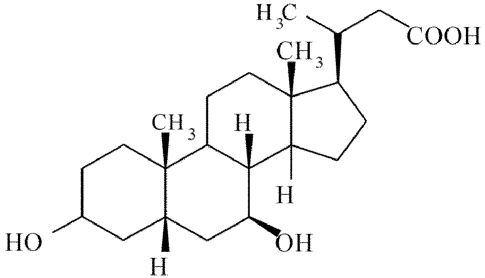

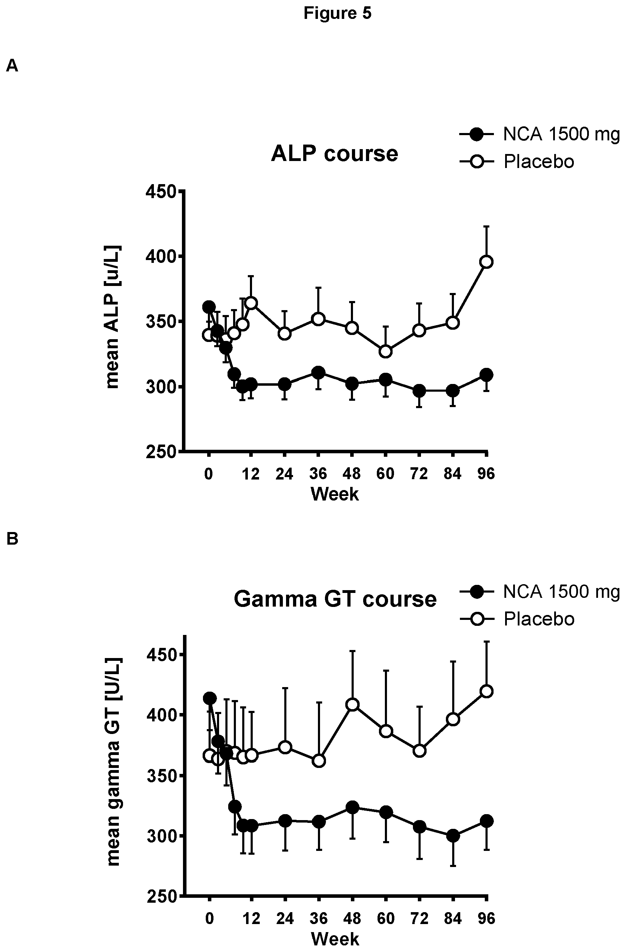

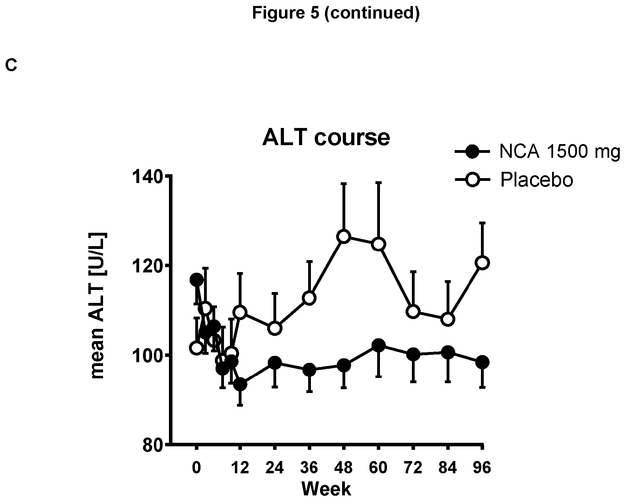

The NCA used in accordance with this invention includes salts, solvates, hydrates, crystal or amorphic forms thereof. Preferably, the NCA is in the form of a crystal prepared as described in WO 2012/072689 A1. In preferred embodiments, a pure polymorph of NCA, or of a pharmaceutically acceptable salt thereof, is used. The polymorph is preferably thermodynamically stable. The polymorph of NCA or pharmaceutically acceptable salt thereof is typically in its anhydrous form. That is, the polymorph crystals contain substantially no water. The amount of water in the crystals is generally less than 1 %, preferably less than 0.5%, more preferably less than 0.1 %, based on the total weight of the crystal. The polymorph is typically characterized by XRPD peaks at 1 1.9, 14.4, 15.3, 15.8, and 16.6 ± 0.2 degrees of 2-theta. Preferably, the polymorph is characterized by the XRPD pattern as shown in Figure 5 for "Form A" of WO 2012/072689 A1. The disclosure of WO 2012/072689 A1 is expressly referred to herein, and its disclosure is incorporated herein in its entirety.

Suitable salts of NCA include, but are not limited to, salts with metal ions such as lithium, aluminum, calcium, magnesium, potassium, sodium, and zinc, and salts with organic bases such as primary amines, secondary amines, tertiary amines, and cyclic amines.

According to the present invention, doses of NCA are expressed as a free base. In other words, even if the considered agent is a pharmaceutically acceptable salt of a small molecule, e.g. the potassium salt of NCA, the doses and daily doses are expressed herein on the bases of the corresponding small molecule free base, e.g. NCA.

The term "patient" or "subject," as used herein, refers to a human subject (e.g. a man, a woman or a child), that has PSC, suffers from PSC, is suspected of having PSC, or is or may be susceptible to a condition caused by and/or associated with PSC, such as Ulcerative colitis, **IBD,** and Crohn's disease.

The human patient to be treated has primary sclerosing cholangitis (PSC). As used herein, "primary sclerosing cholangitis" (PSC) refers to a chronic, progressive, immune-mediated cholestatic liver disease characterized by inflammation, fibrosis, and strictures of the intrahepatic and/or extrahepatic bile ducts, leading to impaired bile flow, hepatocellular injury, and increased risk of cirrhosis and cholangiocarcinoma.

For the purposes of the present invention, a human patient shall be deemed to have PSC, if PSC is confirmed based on fulfillment of at least two (2) of the following diagnostic criteria, with criterion (a) being mandatory unless small-duct PSC is suspected:
(a) Cholangiographic evidence of multifocal bile duct strictures and segmental dilatations (i.e., "beading") observed by magnetic resonance cholangiopancreatography (MRCP) or endoscopic retrograde cholangiopancreatography (ERCP);
(b) Elevated serum levels of one or more cholestatic liver enzymes, including but not limited to alkaline phosphatase (ALP) and/or gamma-glutamyltransferase (GGT), persisting above 1.5 times the upper limit of normal (ULN) on at least two separate occasions within a 3-month period;
(c) Histological findings consistent with PSC, including but not limited to periductal concentric fibrosis ("onion-skin" fibrosis), bile duct injury, and ductopenia, when a liver biopsy is available;
(d) Exclusion of known causes of secondary sclerosing cholangitis, including prior bile duct surgery, bile duct injury, ischemia, infectious cholangitis, or toxin-induced injury;
(e) Association with inflammatory bowel disease (IBD) confirmed by colonoscopy and histopathology, particularly ulcerative colitis or Crohn's colitis, though not required for diagnosis.

Thus, in one embodiment, the present invention is directed to norucholic acid for use in the treatment of primary sclerosing cholangitis in a patient which is a human, wherein
a) the patient has a blood level of serum alkaline phosphatase of ≤ 4.0 times upper limit of normal (ULN), and
b) the patient has a liver stiffness of ≤ 15.0 kPa as measured by an elastometry technique such as vibration-controlled transient elastography (VCTE),

wherein the norucholic acid is administered orally to the patient at a daily dose between 500 mg and 2000 mg,
and wherein PSC is diagnosed in the patient based on fulfillment of at least two (2) of the following diagnostic criteria, with criterion (a) being mandatory unless small-duct PSC is suspected:
   (a) Cholangiographic evidence of multifocal bile duct strictures and segmental dilatations (i.e., "beading") observed by magnetic resonance cholangiopancreatography (MRCP) or endoscopic retrograde cholangiopancreatography (ERCP);
   (b) Elevated serum levels of one or more cholestatic liver enzymes, including but not limited to alkaline phosphatase (ALP) and/or gamma-glutamyltransferase (GGT), persisting above 1.5 times the upper limit of normal (ULN) on at least two separate occasions within a 3-month period;
   (c) Histological findings consistent with PSC, including but not limited to periductal concentric fibrosis ("onion-skin" fibrosis), bile duct injury, and ductopenia, when a liver biopsy is available;
   (d) Exclusion of known causes of secondary sclerosing cholangitis, including prior bile duct surgery, bile duct injury, ischemia, infectious cholangitis, or toxin-induced injury;
   (e) Association with inflammatory bowel disease (IBD) confirmed by colonoscopy and histopathology, particularly ulcerative colitis or Crohn's colitis, though not required for diagnosis.

Thus, the present invention solves the problem to identify, in patients which are diagnosed with PSC in accordance with the above outlined criteria, this group of patients which can 1) successfully be treated with NCA or NCA in combination with UDCA and to 2) sort these patients out which will have no benefit of such a treatment as non-responder and which only would have to suffer from the adverse side effects of the NCA treatment. Intermediate results from an ongoing clinical trial support the conclusion that about 50% of the PSC patients are non-responder. The criteria for being a non-responder as disclosed herein (i.e. blood level of serum alkaline phosphatase of > 4.0 times upper limit of normal and liver stiffness of > 15.0 kPa as measured by vibration-controlled transient elastography (VCTE)) clearly identify the group of the non-responder. However, a patient who does not fulfill these two criteria is not automatically a responder. A person skilled in the art understands that the closer the blood level of serum alkaline phosphatase comes to 4.0 times upper limit of normal and the closer the liver stiffness comes to 15.0 kPa, the more likely it will be that the NCA treatment will not be successful anymore. A patient with a blood level of serum alkaline phosphatase equal to 3.0 times upper limit of normal and a liver stiffness of 13 kPa might still successfully be treated with NCA.

Consequently, the present invention identifies exactly a group of non-responders which is, however, about 50% of all patients, but does not exactly define a group of responders, because it is ethically indefensible not to treat patients which could (but not must) have a benefit from the NCA treatment. Therefore, the group of potential responders is defined more broadly than the group of definite non-responders. Due to the fact that the patients with NCA treatment are monitored continuously, success of the treatment will be assessed. In case of no detectable benefit from the treatment, NCA treatment could be terminated.

As used herein, "alkaline phosphatase" (ALP) refers to a serum enzyme predominantly produced in the liver, bile ducts, and bone, which catalyzes the hydrolysis of phosphate esters in an alkaline environment. In the context of primary sclerosing cholangitis (PSC), elevated serum ALP levels are a biochemical hallmark of cholestasis and reflect impaired bile flow due to inflammation, fibrosis, or strictures of the intrahepatic and/or extrahepatic bile ducts.

ALP can be measured in serum using standard clinical chemistry methods, including colorimetric enzymatic assays or automated analyzer systems routinely employed in clinical laboratories. For the purposes of the present invention, ALP measurement may be performed at baseline (prior to treatment) and at one or more follow-up time points during the course of therapy to monitor treatment response, disease progression, or biochemical normalization. Unless otherwise stated, serum ALP levels are expressed in International Units per Liter (IU/L) and are interpreted in relation to the upper limit of normal (ULN) as defined by the local clinical laboratory reference range. ALP values may be referenced as absolute values or as multiples of ULN.

As used herein, "liver stiffness" refers to a biophysical property of hepatic tissue that reflects its elasticity or rigidity, which changes in response to structural alterations in the liver such as fibrosis, inflammation, cholestasis, or congestion. Liver stiffness increases as fibrotic tissue replaces healthy parenchyma. Elevated liver stiffness values correlate with increasing hepatic fibrosis. Liver stiffness is usually expressed in kilopascals (kPa) and thus provides an objective numerical value.

Liver stiffness may be categorized into fibrosis stages as follows:

| | |
|---|---|
| Normal or no fibrosis (F0-F1): | Liver stiffness < 6.0 kPa |
| Mild to moderate fibrosis (F2): | Liver stiffness approximately 6.0 - 9.5 kPa |
| | Mild: ca. 6.0 - 7.9 kPa |
| | Moderate: ca. 8.0 - 9.5 kPa |
| Advanced fibrosis (F3): | Liver stiffness approximately 9.6 - 14.9 kPa |
| Cirrhosis (F4): | Liver stiffness ≥ 15.0 kPa. |

As used herein, "liver stiffness measurement" (LSM) refers to the non-invasive quantification of liver stiffness, which serves as a surrogate marker for the degree of liver fibrosis. The measurement is typically performed by placing a probe on the patient's skin over the liver, where mechanical or acoustic pulses generate shear waves that propagate through hepatic tissue. The velocity of these waves is directly proportional to tissue stiffness. For the purposes of the present invention, LSM is preferably but not exclusively performed with elastometry techniques selected from the group consisting of: FibroScan^{®}, also known as Vibration-Controlled Transient Elastography (VCTE), Acoustic Radiation Force Impulse (ARFI) imaging, Shear Wave Elastography (SWE), Magnetic Resonance Elastography (MRE), Supersonic Shear Imaging Elastometry, Transient Elastography (TE), and Magnetic Resonance Imaging (MRI)-based stiffness quantification.

Thus, the present invention is directed to norucholic acid (NCA) for use in the treatment of primary sclerosing cholangitis (PSC) in a patient which is a human, wherein
a) the patient has a blood level of serum alkaline phosphatase (ALP) of less than or equal 4.0 times upper limit of normal (ULN) (≤ 4.0 times upper limit of normal), and
b) the patient has a liver stiffness of less than or equal 15.0 kPa (≤ 15.0 kPa) as measured by an elastometry technique such as vibration-controlled transient elastography (VCTE), and
wherein the norucholic acid is administered orally to the patient at a daily dose between 500 mg and 2000 mg.

Unless otherwise specified, it is to be understood that the conditions a) and b) are met at the baseline, i.e. prior treatment begin. In other words, the present invention is directed to norucholic acid (NCA) for use in the treatment of primary sclerosing cholangitis (PSC) in a patient which is a human, wherein
a) the patient has a blood level of serum alkaline phosphatase (ALP) of less than or equal 4.0 times upper limit of normal (ULN) at baseline (≤ 4.0 times upper limit of normal at baseline), and
b) the patient has a liver stiffness of less than or equal 15.0 kPa as measured by an elastometry technique such as vibration-controlled transient elastography (VCTE) at baseline (≤ 15.0 kPa at baseline), and
wherein the norucholic acid is administered orally to the patient at a daily dose between 500 mg and 2000 mg.

In one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of less than or equal 4.0 times upper limit of normal (ULN) at baseline (≤ 4.0 times upper limit of normal at baseline), and
b) the patient has a liver stiffness of less than or equal 15.0 kPa as measured by an elastometry technique such as vibration-controlled transient elastography (VCTE) at baseline (≤ 15.0 kPa at baseline),

wherein the norucholic acid is administered orally to the patient at a daily dose between 500 mg and 2000 mg, and
wherein the patient's blood level of serum alkaline phosphatase reduces below 1.5 times ULN after 12 weeks NCA treatment.

In one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of less than or equal 4.0 times upper limit of normal (ULN) at baseline (≤ 4.0 times upper limit of normal at baseline), and
b) the patient has a liver stiffness of less than or equal 15.0 kPa as measured by an elastometry technique such as vibration-controlled transient elastography (VCTE) at baseline (≤ 15.0 kPa at baseline),

wherein the norucholic acid is administered orally to the patient at a daily dose between 500 mg and 2000 mg, and
wherein the patient's liver stiffness remains unchanged or is reduced after 96 weeks NCA treatment.

In one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of less than or equal 4.0 times upper limit of normal (ULN) at baseline (≤ 4.0 times upper limit of normal at baseline), and
b) the patient has a liver stiffness of less than or equal 15.0 kPa as measured by an elastometry technique such as vibration-controlled transient elastography (VCTE) at baseline (≤ 15.0 kPa at baseline),

wherein the norucholic acid is administered orally to the patient at a daily dose between 500 mg and 2000 mg,
wherein the patient's blood level of serum alkaline phosphatase reduces below 1.5 times ULN after 12 weeks NCA treatment, and
wherein the patient's liver stiffness remains unchanged or is reduced after 96 weeks NCA treatment.

In a preferred embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 times and 4.0 times upper limit of normal (ULN) (≥1.5 and ≤ 4.0 times upper limit of normal), and
b) the patient has a liver stiffness between 6.0 kPa and 15.0 kPa (≥6.0 kPa and ≤ 15.0 kPa) as measured by an elastometry technique such as vibration-controlled transient elastography (VCTE), and
wherein the norucholic acid is administered orally to the patient at a daily dose between 500 mg and 2000 mg.

In a preferred embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 3.5 times upper limit of normal (ULN), and
b) the patient has a liver stiffness of ≤ 15.0 kPa as measured by an elastometry technique such as vibration-controlled transient elastography (VCTE), and
wherein the norucholic acid is administered orally to the patient at a daily dose between 500 mg and 2000 mg.

In a preferred embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 3.0 times upper limit of normal (ULN), and
b) the patient has a liver stiffness of ≤ 15.0 kPa as measured by an elastometry technique such as vibration-controlled transient elastography (VCTE), and
wherein the norucholic acid is administered orally to the patient at a daily dose between 500 mg and 2000 mg.

In a preferred embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 2.5 times upper limit of normal (ULN), and
b) the patient has a liver stiffness of ≤ 15.0 kPa as measured by an elastometry technique such as vibration-controlled transient elastography (VCTE), and
wherein the norucholic acid is administered orally to the patient at a daily dose between 500 mg and 2000 mg.

In a preferred embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 times and 4.0 times upper limit of normal (ULN) (≥ 1.5 and ≤ 4.0 times upper limit of normal), and
b) the patient has a liver stiffness of ≤ 15.0 kPa or between 6.0 kPa and 15.0 kPa, preferably 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg.

In a preferred embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 times and 3.5 times upper limit of normal (ULN) (≥ 1.5 and ≤ 3.5 times upper limit of normal), and
b) the patient has a liver stiffness of ≤ 15.0 kPa or between 6.0 kPa and 15.0 kPa, preferably 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg.

In a preferred embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 times and 3.0 times upper limit of normal (ULN) (≥ 1.5 and ≤ 3.0 times upper limit of normal), and
b) the patient has a liver stiffness of ≤ 15.0 kPa or between 6.0 kPa and 15.0 kPa, preferably 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg.

In a preferred embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 times and 2.5 times upper limit of normal (ULN) (≥ 1.5 and ≤ 2.5 times upper limit of normal), and
b) the patient has a liver stiffness of ≤ 15.0 kPa or between 6.0 kPa and 15.0 kPa, preferably 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg.

In another embodiment of the present invention, the **ELF score** is used to select the patient for treatment with NCA in addition to or as an alternative to liver stiffness. The ELF score is characterized by three biomarker components that together form an extracellular matrix (ECM) marker set comprising (i) a tissue inhibitor of metalloproteinases 1 (TIMP-1), (ii) amino-terminal propeptide of type III procollagen (PIIINP) and (iii) hyaluronic acid (HA), wherein the ELF score correlates to liver function, wherein an ELF score above 11.3 characterizes liver cirrhosis, wherein an ELF score between 9.9 and 11.3 characterizes severe fibrosis, wherein an ELF score between 7.7 and 9.9 characterizes moderate fibrosis, wherein an ELF score below 7.7 characterizes no or weak fibrosis.

Thus, in an alternative embodiment, the present invention is also directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN), and
b') the patient has an Enhanced Liver Fibrosis (ELF) score ≥ 10.0 or the patient does not suffer from severe fibrosis as determined by an ELF Score ≥ 10.0, and
wherein the norucholic acid is administered orally to the patient at a daily dose between 500 mg and 2000 mg.

In an alternative embodiment, the present invention is also directed to norucholic acid (NCA) for use in the treatment of primary sclerosing cholangitis (PSC) in a patient which is a human, wherein
a) the patient has a blood level of serum alkaline phosphatase (ALP) between 1.5 times and 4.0 times upper limit of normal (ULN) (≥1.5 and ≤ 4.0 times upper limit of normal), and
b') the patient has an Enhanced Liver Fibrosis (ELF) score between 10.0 and 12.0, and
wherein the norucholic acid is administered orally to the patient at a daily dose between 500 mg and 2000 mg.

Preferably, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN), and
b') the patient does not suffer from severe fibrosis as determined by the ELF Score ≥ 10,
   or
a) the patient has a blood level of serum alkaline phosphatase (ALP) between 1.5 times and 4.0 times upper limit of normal (ULN) (≥1.5 and ≤ 4.0 times upper limit of normal), and
b') the patient has an Enhanced Liver Fibrosis (ELF) score between 10.0 and 12.0,
and wherein the norucholic acid is administered orally to the patient at a daily dose between 500 mg and 2000 mg and
wherein the ELF Score is characterized by three biomarker components that together form an extracellular matrix (ECM) marker set comprising (i) a tissue inhibitor of metalloproteinases 1 (TIMP-1), (ii) amino-terminal propeptide of type III procollagen (PIIINP) and (iii) hyaluronic acid (HA), wherein the ELF score correlates to liver function, wherein an ELF score above 11.3 characterizes liver cirrhosis, wherein an ELF score between 9.9 and 11.3 characterizes severe fibrosis, wherein an ELF score between 7.7 and 9.9 characterizes moderate fibrosis, wherein an ELF score below 7.7 characterizes no or weak fibrosis.

In an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 times and 3.5 or 3.0 or 2.5 times upper limit of normal (ULN), and
b') the patient does not suffer from severe fibrosis as determined by the ELF Score ≥ 10.0, and
wherein the norucholic acid is administered orally to the patient at a daily dose between 500 mg and 2000 mg.

**In** an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient does not suffer from severe fibrosis as determined by the ELF Score ≥ 10.0, and
wherein the norucholic acid is administered orally to the patient at a daily dose between 500 mg and 2000 mg.

Preferably, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa as measured by an elastometry technique such as vibration-controlled transient elastography (VCTE), and
c) the patient does not suffer from severe fibrosis as determined by the **ELF** Score ≥ 10.0,

wherein the norucholic acid is administered orally to the patient at a daily dose between 500 mg and 2000 mg, and
wherein the **ELF** Score is characterized by three biomarker components that together form an extracellular matrix (ECM) marker set comprising (i) a tissue inhibitor of metalloproteinases 1 (TIMP-1), (ii) amino-terminal propeptide of type III procollagen (PIIINP) and (iii) hyaluronic acid (HA), wherein the ELF score correlates to liver function, wherein an ELF score above 11.3 characterizes liver cirrhosis, wherein an ELF score between 9.9 and 11.3 characterizes severe fibrosis, wherein an ELF score between 7.7 and 9.9 characterizes moderate fibrosis, wherein an ELF score below 7.7 characterizes no or weak fibrosis.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10, and
wherein the norucholic acid is administered orally to the patient at a daily dose between 500 mg and 2000 mg.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN), and
b) the patient has a liver stiffness of between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score ≥ 10.0, and
wherein the norucholic acid is administered orally to the patient at a daily dose between 500 mg and 2000 mg.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN), and
b) the patient has a liver stiffness of between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0, and
wherein the norucholic acid is administered orally to the patient at a daily dose between 500 mg and 2000 mg.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score between 10.0 and 12.0, and
wherein the norucholic acid is administered orally to the patient at a daily dose between 500 mg and 2000 mg.

Preferably, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN), and
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0,

wherein the norucholic acid is administered orally to the patient at a daily dose between 500 mg and 2000 mg, and
wherein the ELF Score is characterized by three biomarker components that together form an extracellular matrix (ECM) marker set comprising (i) a tissue inhibitor of metalloproteinases 1 (TIMP-1), (ii) amino-terminal propeptide of type III procollagen (PIIINP) and (iii) hyaluronic acid (HA), wherein the ELF score correlates to liver function, wherein an ELF score above 11.3 characterizes liver cirrhosis, wherein an ELF score between 9.9 and 11.3 characterizes severe fibrosis, wherein an ELF score between 7.7 and 9.9 characterizes moderate fibrosis, wherein an ELF score below 7.7 characterizes no or weak fibrosis.

Preferably, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN), and
b) the patient has a liver stiffness of between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,

wherein the norucholic acid is administered orally to the patient at a daily dose between 500 mg and 2000 mg, and
wherein the ELF Score is characterized by three biomarker components that together form an extracellular matrix (ECM) marker set comprising (i) a tissue inhibitor of metalloproteinases 1 (TIMP-1), (ii) amino-terminal propeptide of type III procollagen (PIIINP) and (iii) hyaluronic acid (HA), wherein the ELF score correlates to liver function, wherein an ELF score above 11.3 characterizes liver cirrhosis, wherein an ELF score between 9.9 and 11.3 characterizes severe fibrosis, wherein an ELF score between 7.7 and 9.9 characterizes moderate fibrosis, wherein an ELF score below 7.7 characterizes no or weak fibrosis.

In another embodiment of the present invention, the absence of liver cirrhosis is used to select the patient for treatment with NCA in addition to or as an alternative to liver stiffness. As used herein, "liver cirrhosis with a Child-Pugh classification of ≥9'' refers to clinically advanced hepatic cirrhosis, representing Child-Pugh class B or C, as defined by the Child-Pugh scoring system. This classification is based on the evaluation of five clinical and laboratory parameters: serum bilirubin, serum albumin, prothrombin time (or INR), presence and severity of ascites, and hepatic encephalopathy. Each parameter is assigned a score from 1 to 3 points, with a total score range of 5 to 15. A score of ≥9 points indicates decompensated cirrhosis, associated with impaired hepatic reserve and a significantly increased risk of liver-related complications.

For the purposes of the present invention, a subject is considered to have liver cirrhosis with Child-Pugh ≥9 if they meet the Child-Pugh total score equal to or greater than 9, which corresponds to Child-Pugh class B (score 7-9) or class C (score 10-15), and reflects moderate to severe hepatic dysfunction, with clinical signs such as ascites, coagulopathy, jaundice, or encephalopathy. Patients without such cirrhosis, i.e., those with Child-Pugh scores less than 9 (Child-Pugh class A or early B), are considered to have compensated liver function and are therefore deemed eligible responders to the NCA treatment described herein.

Thus, in an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN), and
b") the patient does not suffer from liver cirrhosis which is diagnosed or classified by a Child classification of ≥ 9 or ≥ 9 - 15, and
wherein the norucholic acid is administered orally to the patient at a daily dose between 500 mg and 2000 mg.

In an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN), and
b") the patient has a Child classification of < 9 or between 5 and 9, preferably between 5 and 7, and
wherein the norucholic acid is administered orally to the patient at a daily dose between 500 mg and 2000 mg.

In an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN), and
b‴) the patient does not suffer from hepatic encephalopathy and from liver cirrhosis
   which are diagnosed or classified by a Child classification of ≥ 9 or ≥ 9 - 15,
and wherein the norucholic acid is administered orally to the patient at a daily dose between 500 mg and 2000 mg.

In an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN), and
b‴) the patient has a Child classification of < 9 or between 5 and 9, preferably between 5 and 7, and
wherein the norucholic acid is administered orally to the patient at a daily dose between 500 mg and 2000 mg.

In an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient does not suffer from liver cirrhosis which is diagnosed or classified by a Child classification of ≥ 9 or ≥ 9 - 15, and
wherein the norucholic acid is administered orally to the patient at a daily dose between 500 mg and 2000 mg.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has a Child classification of < 9 or between 5 and 9, preferably between 5 and 7, and
wherein the norucholic acid is administered orally to the patient at a daily dose between 500 mg and 2000 mg.

In a preferred embodiment, NCA is administered orally daily to the patient for at least at least 12 weeks, more preferably at least 16 weeks, more preferably at least 20 weeks, more preferably at least 24 weeks, more preferably at least 30 weeks, more preferably at least 36 weeks, more preferably at least 44 weeks, more preferably at least 52 weeks, more preferably at least 60 weeks, more preferably at least 72 weeks, more preferably at least 84 weeks, and most preferably at least 96 weeks.

Thus, in a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
wherein the norucholic acid is administered orally to the patient at a daily dose between 500 mg and 2000 mg for at least 12 weeks, preferably at least 16 weeks, more preferably at least 20 weeks, more preferably at least 24 weeks, more preferably at least 30 weeks, more preferably at least 36 weeks, more preferably at least 44 weeks, more preferably at least 52 weeks, more preferably at least 60 weeks, more preferably at least 72 weeks, more preferably at least 84 weeks, and most preferably at least 96 weeks.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg for at least 12 weeks, preferably at least 16 weeks, more preferably at least 20 weeks, more preferably at least 24 weeks, more preferably at least 30 weeks, more preferably at least 36 weeks, more preferably at least 44 weeks, more preferably at least 52 weeks, more preferably at least 60 weeks, more preferably at least 72 weeks, more preferably at least 84 weeks, and most preferably at least 96 weeks.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥10.0, and
wherein the norucholic acid is administered orally to the patient at a daily dose between 500 mg and 2000 mg for at least 12 weeks, preferably at least 16 weeks, more preferably at least 20 weeks, more preferably at least 24 weeks, more preferably at least 30 weeks, more preferably at least 36 weeks, more preferably at least 44 weeks, more preferably at least 52 weeks, more preferably at least 60 weeks, more preferably at least 72 weeks, more preferably at least 84 weeks, and most preferably at least 96 weeks.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,
wherein the norucholic acid is administered orally to the patient at a daily dose between 500 mg and 2000 mg for at least 12 weeks, preferably at least 16 weeks, more preferably at least 20 weeks, more preferably at least 24 weeks, more preferably at least 30 weeks, more preferably at least 36 weeks, more preferably at least 44 weeks, more preferably at least 52 weeks, more preferably at least 60 weeks, more preferably at least 72 weeks, more preferably at least 84 weeks, and most preferably at least 96 weeks.

In a preferred embodiment, NCA is administered orally daily to the patient for at least 96 weeks. Thus, in a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
wherein the norucholic acid is administered orally to the patient at a daily dose between 500 mg and 2000 mg for at least 96 weeks.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg for at least 96 weeks.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and
wherein the norucholic acid is administered orally to the patient at a daily dose between 500 mg and 2000 mg for at least 96 weeks.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,
wherein the norucholic acid is administered orally to the patient at a daily dose between 500 mg and 2000 mg for at least 96 weeks.

The inventors have further found that patients treated with one or more medications selected from: a glucocorticoid, an immunosuppressive drug, chlorambucil, pentoxifylline, penicillamine, pirfenidone, rifampicin, an integrin-antagonist or an anti-tumor necrosis factor-α for at least 3 months prior to the start of norucholic acid treatment do not respond to the NCA treatment according to the present invention. Thus, in another embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE),

wherein the norucholic acid is administered orally to the patient at a daily dose between 500 mg and 2000 mg, and
wherein the patient is not treated with one or more medications selected from: a glucocorticoid, an immunosuppressive drug, chlorambucil, pentoxifylline, penicillamine, pirfenidone, rifampicin, an integrin-antagonist or an anti-tumor necrosis factor-α for at least 3 months prior to the start of norucholic acid treatment.

Preferably, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg, and
wherein the patient is not treated with one or more medications selected from: a glucocorticoid, an immunosuppressive drug, chlorambucil, pentoxifylline, penicillamine, pirfenidone, rifampicin, an integrin-antagonist or an anti-tumor necrosis factor-α for at least 3 months prior to the start of norucholic acid treatment.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and

wherein the norucholic acid is administered orally to the patient at a daily dose between 500 mg and 2000 mg, and
wherein the patient is not treated with one or more medications selected from: a glucocorticoid, an immunosuppressive drug, chlorambucil, pentoxifylline, penicillamine, pirfenidone, rifampicin, an integrin-antagonist or an anti-tumor necrosis factor-α for at least 3 months prior to the start of norucholic acid treatment.

**In** a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,

wherein the norucholic acid is administered orally to the patient at a daily dose between 500 mg and 2000 mg, and
wherein the patient is not treated with one or more medications selected from: a glucocorticoid, an immunosuppressive drug, chlorambucil, pentoxifylline, penicillamine, pirfenidone, rifampicin, an integrin-antagonist or an anti-tumor necrosis factor-α for at least 3 months prior to the start of norucholic acid treatment.

Preferably, the glucocorticoid is selected from Hydrocortisone, Cortisone, Prednisone, Prednisolone, Methylprednisolone, Dexamethasone, Betamethasone, Triamcinolone, Deflazacort, Budesonide, Beclometasone, Fluticasone, Mometasone, Ciclesonide, Alclometasone, Clobetasol, Clobetasone, Clocortolone, Desonide, Desoximetasone, Diflorasone, Diflucortolone, Fluocinolone, Fluocinonide, Fluocortolone, Flurandrenolide, Flumetasone, Flunisolide, Fluorometholone, Halcinonide, Halobetasol, Halometasone, Loteprednol, Meprednisone, Paramethasone, Prednicarbate, Tixocortol, Amcinonide, Cloprednol, Fluprednisolone, Fluprednidene, Rimexolone, Vamorolone, Cortivazol, Difluprednate, Fluocortin, Fluperolone, Fluorandrenolone, Fluocortinbutyl, Fluocortolone caproate, Fluocortolone pivalate, Fluocortolone hexanoate, Fluocortolone acetate, Fluocortolone butyrate, Fluocortolone valerate, Fluocortolone benzoate, and Fluocortolone propionate

Preferably, the immunosuppressive drug is selected from Cyclosporine, Tacrolimus, Pimecrolimus, Sirolimus (Rapamycin), Everolimus, Temsirolimus, Zotarolimus, Azathioprine, 6-Mercaptopurine, Methotrexate, Mycophenolate mofetil, Mycophenolic acid, Leflunomide, Cyclophosphamide, Chlorambucil, Melphalan, Rituximab, Infliximab, Adalimumab, Etanercept, Golimumab, Certolizumab pegol, Basiliximab, Daclizumab, Tocilizumab, Ustekinumab, Secukinumab, Ixekizumab, Brodalumab, Belatacept, Abatacept, Alemtuzumab, Eculizumab, Anifrolumab, Tofacitinib, Baricitinib, Upadacitinib, Filgotinib, Fingolimod, Siponimod, Ozanimod, Ponesimod, Thalidomide, Lenalidomide, Bortezomib, Hydroxychloroquine, Sulfasalazine, Colchicine, Dapsone, Penicillamine, Dimethyl fumarate, Monomethyl fumarate, and Teriflunomide.

Preferably, the integrin-antagonist is selected from Natalizumab, Vedolizumab, Etrolizumab, Abrilumab, Carotegrast methyl (also known as AJM300), PF-00547659, Abciximab, Eptifibatide, Tirofiban, Cilengitide, Volociximab, Firategrast, EMD527040, TBC4746, IVL745, GW559090, and QLT0267.

Preferably, the anti-tumor necrosis factor-α is selected from Adalimumab, Adalimumab-atto, Adalimumab-adbm, Adalimumab-adaz, Adalimumab-afzb, Adalimumab-aqvh, Adalimumab-aaty, Adalimumab-yhzh, Adalimumab-aacf, Adalimumab-ryvk, Etanercept, Etanercept-szzs, Infliximab, Infliximab-abda, Infliximab-dyyb, Infliximab-axxq, Infliximab-qbtx, Certolizumab pegol, Golimumab, Afelimomab, Onercept, Pegsunercept, and Lenercept.

The inventors have further identified that patients having elevated serum levels of one or both of alanine aminotransferase (ALT) and aspartate aminotransferase (AST) at baseline respond particularly well to the NCA treatment according to the invention.

Preferably, the patient's serum levels of one or both of alanine aminotransferase (ALT) and/or aspartate aminotransferase (AST) at baseline are elevated by 1.1 ≥ times ULN. Preferably, the patient's serum levels of one or more of alanine aminotransferase (ALT) or aspartate aminotransferase (AST) at baseline are elevated by 1.2 ≥ times ULN. Preferably, the patient's serum levels of one or more of alanine aminotransferase (ALT) or aspartate aminotransferase (AST) at baseline are elevated by 1.3 ≥ times ULN. Preferably, the patient's serum levels of one or more of alanine aminotransferase (ALT) or aspartate aminotransferase (AST) at baseline are elevated by 1.4 ≥ times ULN. Preferably, the patient's serum levels of one or more of alanine aminotransferase (ALT) or aspartate aminotransferase (AST) at baseline are elevated by 1.5 ≥ times ULN.

Thus, in one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally to the patient at a daily dose between 500 mg and 2000 mg, and
wherein the patient has serum levels ≥ 1.5 times upper limit of normal of one or both of alanine aminotransferase (ALT) or aspartate aminotransferase (AST).

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg and
wherein the patient has serum levels ≥ 1.5 times upper limit of normal of one or both of alanine aminotransferase (ALT) or aspartate aminotransferase (AST).

**In** a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and

wherein the norucholic acid is administered orally to the patient at a daily dose between 500 mg and 2000 mg and
wherein the patient has serum levels ≥ 1.5 times upper limit of normal of one or both of alanine aminotransferase (ALT) or aspartate aminotransferase (AST).

**In** a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,

wherein the norucholic acid is administered orally to the patient at a daily dose between 500 mg and 2000 mg and
wherein the patient has serum levels ≥ 1.5 times upper limit of normal of one or both of alanine aminotransferase (ALT) or aspartate aminotransferase (AST).

**In** a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
wherein the norucholic acid is administered orally to the patient at a daily dose between 500 mg and 2000 mg for at least 12 weeks, preferably for at least 96 weeks and wherein the patient has serum levels ≥ 1.5 times upper limit of normal of one or both of alanine aminotransferase (ALT) or aspartate aminotransferase (AST).

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg for at least 12 weeks, preferably for at least 96 weeks and wherein the patient has serum levels ≥ 1.5 times upper limit of normal of one or both of alanine aminotransferase (ALT) or aspartate aminotransferase (AST).

The inventors have further found that patient suffering from large duct cholangitis respond particularly well to the NCA treatment according to the invention. Thus, in one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0, preferably ≤ 12.5 kPa as measured by an elastometry technique such as vibration-controlled transient elastography (VCTE), and

wherein the norucholic acid is administered orally to the patient at a daily dose between 500 mg and 2000 mg, and
wherein the patient suffers from large duct cholangitis.

**In** a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg and
wherein the patient suffers from large duct cholangitis.

**In** one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally to the patient at a daily dose between 500 mg and 2000 mg,
wherein the patient suffers from large duct cholangitis and
wherein the patient has serum levels ≥ 1.5 times upper limit of normal of one or both of alanine aminotransferase (ALT) or aspartate aminotransferase (AST).

**In** a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN), and
b) the patient has a liver stiffness of between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg,
wherein the patient suffers from large duct cholangitis and
wherein the patient has serum levels ≥ 1.5 times upper limit of normal of one or both of alanine aminotransferase (ALT) or aspartate aminotransferase (AST).

**In** a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and

wherein the norucholic acid is administered orally to the patient at a daily dose between 500 mg and 2000 mg and
wherein the patient suffers from large duct cholangitis.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,

wherein the norucholic acid is administered orally to the patient at a daily dose between 500 mg and 2000 mg and
wherein the patient suffers from large duct cholangitis.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
wherein the norucholic acid is administered orally to the patient at a daily dose between 500 mg and 2000 mg for at least 12 weeks, preferably for at least 96 weeks and wherein the patient suffers from large duct cholangitis.

In an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient does not suffer from liver cirrhosis which is diagnosed or classified by a Child classification of ≥ 9 or ≥ 9 - 15, and

wherein the norucholic acid is administered orally to the patient at a daily dose between 500 mg and 2000 mg and
wherein the patient has serum levels ≥ 1.5 times upper limit of normal of one or both of alanine aminotransferase (ALT) or aspartate aminotransferase (AST).

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has a Child classification of < 9 or between 5 and 9, preferably between 5 and 7, and

wherein the norucholic acid is administered orally to the patient at a daily dose between 500 mg and 2000 mg and
wherein the patient has serum levels ≥ 1.5 times upper limit of normal of one or both of alanine aminotransferase (ALT) or aspartate aminotransferase (AST).

In an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and
d) the patient does not suffer from liver cirrhosis which is diagnosed or classified by a Child classification of ≥ 9 or ≥ 9 - 15, and
wherein the norucholic acid is administered orally to the patient at a daily dose between 500 mg and 2000 mg.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,
d) the patient has a Child classification of < 9 or between 5 and 9, preferably between 5 and 7, and
wherein the norucholic acid is administered orally to the patient at a daily dose between 500 mg and 2000 mg.

**In** an alternative embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and
d) the patient does not suffer from liver cirrhosis which is diagnosed or classified by a Child classification of ≥ 9 or ≥ 9 - 15, and

wherein the norucholic acid is administered orally to the patient at a daily dose between 500 mg and 2000 mg and
wherein the patient has serum levels ≥ 1.5 times upper limit of normal of one or both of alanine aminotransferase (ALT) or aspartate aminotransferase (AST).

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,
d) the patient has a Child classification of < 9 or between 5 and 9, preferably between 5 and 7, and

wherein the norucholic acid is administered orally to the patient at a daily dose between 500 mg and 2000 mg and
wherein the patient has serum levels ≥ 1.5 times upper limit of normal of one or both of alanine aminotransferase (ALT) or aspartate aminotransferase (AST).

The inventors have further found that patients exhibiting positive anti-nuclear antibody (ANA) and/or perinuclear anti-neutrophil cytoplasmic antibody (pANCA) serology, and negative antimitochondrial antibody (AMA) serology respond particularly well to the NCA treatment according to the invention. Thus, in one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally to the patient at a daily dose between 500 mg and 2000 mg, and
wherein the patient exhibits positive anti-nuclear antibody (ANA) and/or perinuclear anti-neutrophil cytoplasmic antibody (pANCA) serology, and negative antimitochondrial antibody (AMA) serology.

The present invention is also directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg, and
wherein the patient exhibits positive anti-nuclear antibody (ANA) and/or perinuclear anti-neutrophil cytoplasmic antibody (pANCA) serology, and negative antimitochondrial antibody (AMA) serology.

In one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally to the patient at a daily dose between 500 mg and 2000 mg, and
wherein the patient exhibits positive anti-nuclear antibody (ANA).

A further embodiment of the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg, and
wherein the patient exhibits positive anti-nuclear antibody (ANA).

In one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally to the patient at a daily dose between 500 mg and 2000 mg, and
wherein the patient exhibits positive perinuclear anti-neutrophil cytoplasmic antibody (pANCA) serology.

A further embodiment of the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg, and
wherein the patient exhibits positive perinuclear anti-neutrophil cytoplasmic antibody (pANCA) serology.

In one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally to the patient at a daily dose between 500 mg and 2000 mg, and
wherein the patient exhibits negative antimitochondrial antibody (AMA) serology.

A further embodiment of the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg, and
wherein the patient exhibits negative antimitochondrial antibody (AMA) serology.

In one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally to the patient at a daily dose between 500 mg and 2000 mg, and
wherein the patient exhibits positive anti-nuclear antibody (ANA) and positive perinuclear anti-neutrophil cytoplasmic antibody (pANCA) serology and negative antimitochondrial antibody (AMA) serology.

A further embodiment of the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg, and
wherein the patient exhibits positive anti-nuclear antibody (ANA) and positive perinuclear anti-neutrophil cytoplasmic antibody (pANCA) serology and negative antimitochondrial antibody (AMA) serology.

In one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally to the patient at a daily dose between 500 mg and 2000 mg,
wherein the patient suffers from large duct cholangitis and
wherein the patient exhibits positive anti-nuclear antibody (ANA) and/or perinuclear anti-neutrophil cytoplasmic antibody (pANCA) serology, and negative antimitochondrial antibody (AMA) serology.

A further embodiment of the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg, and
wherein the patient suffers from large duct cholangitis and
wherein the patient exhibits positive anti-nuclear antibody (ANA) and/or perinuclear anti-neutrophil cytoplasmic antibody (pANCA) serology, and negative antimitochondrial antibody (AMA) serology.

In one embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally to the patient at a daily dose between 500 mg and 2000 mg,
wherein the patient exhibits positive anti-nuclear antibody (ANA) and/or perinuclear anti-neutrophil cytoplasmic antibody (pANCA) serology, and negative antimitochondrial antibody (AMA) serology and
wherein the patient has elevated serum levels of one or more of alanine aminotransferase (ALT) or aspartate aminotransferase (AST) at baseline.

A further embodiment of the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally to the patient at a daily dose between 500 mg and 2000 mg, and
wherein the patient exhibits positive anti-nuclear antibody (ANA) and/or perinuclear anti-neutrophil cytoplasmic antibody (pANCA) serology, and negative antimitochondrial antibody (AMA) serology and
wherein the patient has elevated serum levels of one or more of alanine aminotransferase (ALT) or aspartate aminotransferase (AST) at baseline.

Preferably, the patient's serum levels of one or both of alanine aminotransferase (ALT) and/or aspartate aminotransferase (AST) at baseline are elevated by 1.1 ≥ times ULN. Preferably, the patient's serum levels of one or more of alanine aminotransferase (ALT) or aspartate aminotransferase (AST) at baseline are elevated by 1.2 ≥ times ULN. Preferably, the patient's serum levels of one or more of alanine aminotransferase (ALT) or aspartate aminotransferase (AST) at baseline are elevated by 1.3 ≥ times ULN. Preferably, the patient's serum levels of one or more of alanine aminotransferase (ALT) or aspartate aminotransferase (AST) at baseline are elevated by 1.4 ≥ times ULN. Preferably, the patient's serum levels of one or more of alanine aminotransferase (ALT) or aspartate aminotransferase (AST) at baseline are elevated by 1.5 ≥ times ULN.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and

wherein the norucholic acid is administered orally to the patient at a daily dose between 500 mg and 2000 mg and
wherein the patient exhibits positive anti-nuclear antibody (ANA) and/or perinuclear anti-neutrophil cytoplasmic antibody (pANCA) serology, and negative antimitochondrial antibody (AMA) serology.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,

wherein the norucholic acid is administered orally to the patient at a daily dose between 500 mg and 2000 mg and
wherein the patient exhibits positive anti-nuclear antibody (ANA) and/or perinuclear anti-neutrophil cytoplasmic antibody (pANCA) serology, and negative antimitochondrial antibody (AMA) serology.

In a further embodiment, the present invention is directed to norucholic acid (NCA) for use in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
wherein the norucholic acid is administered orally to the patient at a daily dose between 500 mg and 2000 mg for at least 12 weeks, preferably for at least 96 weeks and wherein the patient exhibits positive anti-nuclear antibody (ANA) and/or perinuclear anti-neutrophil cytoplasmic antibody (pANCA) serology, and negative antimitochondrial antibody (AMA) serology.

A further embodiment of the present invention is directed to norucholic acid (NCA) for use in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg for at least 12 weeks, preferably for at least 96 weeks and wherein the patient exhibits positive anti-nuclear antibody (ANA) and/or perinuclear anti-neutrophil cytoplasmic antibody (pANCA) serology, and negative antimitochondrial antibody (AMA) serology.

### Pharmaceutical composition

The norucholic acid is typically administered in the form of a pharmaceutical composition which may comprise one or more suitable excipients which are pharmaceutically acceptable.

The norucholic acid can be formulated for oral administration, wherein these formulations further comprise pharmaceutically acceptable carriers, adjuvants, excipients and/or vehicles. Solid dosage forms for oral administration can include tablets, capsules, pills, powders and granules. In such solid dosage forms, the NCA can be admixed with regularly used substances like sucrose, mannitol, sorbitol, starch and starch derivatives, cellulose and cellulose derivates (e.g., microcrystalline cellulose), di-calcium phosphate, lactose, colloidal anhydrous silica, talc, lubricating agents (e.g. magnesium stearate, macrogols), disintegrants and buffering agents. Tablets and pills can also be prepared with enteric coatings in order to prevent that NCA is affected by the stomach acids and enzymes.

The dosage forms comprising the NCA can further include conventional excipients, preferably pharmaceutically acceptable organic or inorganic carrier substances which do not react with the active compound. Suitable pharmaceutically acceptable carriers include, for instance, water, salt solutions, alcohol, oils, preferably vegetable oils, polyethylene glycols, gelatin, lactose, amylose, magnesium stearate, surfactants, perfume oil, fatty acid mono- glycerides and diglycerides, petroethral fatty acid esters, hydroxymethyl-cellulose, polyvinylpyrrolidone and the like. The pharmaceutical preparations can be sterilized and if desired, mixed with auxiliary agents, like lubricants, preservatives, stabilizers, wetting agents, emulsifiers, salts for influencing osmotic pressure, buffers, colorings, flavouring and/or aromatic substances and the like which do not deleteriously react with the active compounds. For parenteral application, particularly suitable vehicles consist of solutions, preferably oily or aqueous solutions, as well as suspensions, emulsions, or implants.

Various delivery systems are known and can be used to administer the NCA, including, for example, encapsulation in liposomes, emulsions, microparticles, microcapsules and microgranules. The required dosage can be administered as a single unit or in a sustained release form. The required dosage form may further be administered in a multiple unit dosage form, in immediate, sustained, prolonged, or extended release form, prepared by coating, as a matrix formulation and the like. The bioavailability of the NCA may be enhanced by micronization of the formulations using conventional techniques such as grinding, milling and spray drying in the presence of suitable excipients or agents such as phospholipids or surfactants.

The NCA may be formulated in a pharmaceutically acceptable salt form. Pharmaceutically acceptable salts of NCA include preferably metal salts, in particular alkali metal salts, or other pharmaceutically acceptable salts. Pharmaceutically acceptable base addition salts include metallic salts made from lithium, calcium, magnesium, potassium, sodium and zinc or organic salts made from primary, secondary and tertiary amines and cyclic amines.

The pharmaceutical composition comprises preferably an effective amount of NCA and a pharmaceutically acceptable carrier and/or excipient.

The daily dose of NCA is between 500 mg and 2000 mg. Preferably, the daily dose of NCA is between 600 mg and 1900 mg, more preferably between 700 mg and 1800 mg and most preferably between 750 mg and 1750 mg. Preferably, the daily dose of NCA is selected from 500 mg, 600 mg, 700 mg, 800 mg, 900 mg, 1000 mg, 1100 mg, 1200 mg, 1300 mg, 1400 mg, 1500 mg, 1600 mg, 1700 mg, 1800 mg, 1900 mg, and 2000 mg. Said amounts are administered preferably at once or possibly in more than one dose (at least 2, 3, 4, 5 or 10 doses) per day. The drug or the pharmaceutical composition may be administered for at least 12 weeks, preferably at least 16 weeks, more preferably at least 20 weeks, more preferably at least 24 weeks, more preferably at least 30 weeks, more preferably at least 36 weeks, more preferably at least 44 weeks, more preferably at least 52 weeks, more preferably at least 60 weeks, more preferably at least 72 weeks, more preferably at least 84 weeks, and most preferably at least 96 weeks, in particular until resolution of PSC.

### Combination therapy

NCA or salts thereof can be administered not only in combination with pharmaceutically acceptable carriers and in dosage forms as described herein, but, of course, also in combination with UDCA (ursodeoxycholic acid) and/or at least one antifibrotic agent.

The combination treatment in accordance with the present invention may comprise administering the active agents in admixture or as separate dosage forms. The combination treatment may comprise administering the active agents simultaneously or in a timely separated manner, e.g. sequentially. The treatment may comprise administering at least one antifibrotic agent.

Thus in one embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
wherein the norucholic acid is administered orally to the patient at a daily dose between 500 mg and 2000 mg.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and
wherein the norucholic acid is administered orally to the patient at a daily dose between 500 mg and 2000 mg.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,
wherein the norucholic acid is administered orally to the patient at a daily dose between 500 mg and 2000 mg.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
wherein the norucholic acid is administered orally to the patient at a daily dose between 500 mg and 2000 mg for at least 12 weeks, preferably for at least 96 weeks.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg for at least 12 weeks, preferably for at least 96 weeks.

In one embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
wherein the norucholic acid is administered orally to the patient at a daily dose between 500 mg and 2000 mg and the ursodeoxycholic acid (UDCA) is orally daily concurrently administered at a dose of 13 - 23 mg/kg/day, preferably 15 - 20 mg/kg/day.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
wherein the norucholic acid is administered orally to the patient at a daily dose between 500 mg and 2000 mg and the ursodeoxycholic acid (UDCA) is orally daily concurrently administered at a dose of 13 - 23 mg/kg/day, preferably 15 - 20 mg/kg/day.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and
wherein the norucholic acid is administered orally to the patient at a daily dose between 500 mg and 2000 mg and the ursodeoxycholic acid (UDCA) is orally daily concurrently administered at a dose of 13 - 23 mg/kg/day, preferably 15 - 20 mg/kg/day.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,
wherein the norucholic acid is administered orally to the patient at a daily dose between 500 mg and 2000 mg and the ursodeoxycholic acid (UDCA) is orally daily concurrently administered at a dose of 13 - 23 mg/kg/day, preferably 15 - 20 mg/kg/day.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient does not suffer from liver cirrhosis which is diagnosed or classified by a Child classification of ≥ 9 or ≥ 9 - 15, and
wherein the norucholic acid is administered orally to the patient at a daily dose between 500 mg and 2000 mg and the ursodeoxycholic acid (UDCA) is orally daily concurrently administered at a dose of 13 - 23 mg/kg/day, preferably 15 - 20 mg/kg/day.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has a Child classification of < 9 or between 5 and 9, preferably between 5 and 7, and
wherein the norucholic acid is administered orally to the patient at a daily dose between 500 mg and 2000 mg and the ursodeoxycholic acid (UDCA) is orally daily concurrently administered at a dose of 13 - 23 mg/kg/day, preferably 15 - 20 mg/kg/day.

In a further embodiment, norucholic acid is administered daily once in the morning and ursodeoxycholic acid (UDCA) is administered daily once in the evening. In particular, the administrations of norucholic acid and ursodeoxycholic acid (UDCA) are between 6 - 10 hours, preferably 8 hours, apart from each other.

In one embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the norucholic acid is administered orally daily once in the morning to the patient at a dose between 500 mg and 2000 mg and the ursodeoxycholic acid (UDCA) is administered orally daily once in the evening at a dose of 13 - 23 mg/kg/day, preferably 15 - 20 mg/kg/day or
the oral daily administrations of norucholic acid at a dose between 500 mg and 2000 mg and ursodeoxycholic acid at a dose of 13 - 23 mg/kg/day, preferably 15 - 20 mg/kg/day are between 6 - 10 hours, preferably 8 hours, apart from each other.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the norucholic acid is administered orally daily once in the morning to the patient at a dose between 500 mg and 2000 mg and the ursodeoxycholic acid (UDCA) is administered orally daily once in the evening at a dose of 13 - 23 mg/kg/day, preferably 15 - 20 mg/kg/day or
the oral daily administrations of norucholic acid at a dose between 500 mg and 2000 mg and ursodeoxycholic acid at a dose of 13 - 23 mg/kg/day, preferably 15 - 20 mg/kg/day are between 6 - 10 hours, preferably 8 hours, apart from each other.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and

wherein the norucholic acid is administered orally daily once in the morning to the patient at a dose between 500 mg and 2000 mg and the ursodeoxycholic acid (UDCA) is administered orally daily once in the evening at a dose of 13 - 23 mg/kg/day, preferably 15 - 20 mg/kg/day or
the oral daily administrations of norucholic acid at a dose between 500 mg and 2000 mg and ursodeoxycholic acid at a dose of 13 - 23 mg/kg/day, preferably 15 - 20 mg/kg/day are between 6 - 10 hours, preferably 8 hours, apart from each other.

In a further embodiment, the present invention is directed to norucholic acid (NCA) for use in combination with UDCA (ursodeoxycholic acid) in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,

wherein the norucholic acid is administered orally daily once in the morning to the patient at a dose between 500 mg and 2000 mg and the ursodeoxycholic acid (UDCA) is administered orally daily once in the evening at a dose of 13 - 23 mg/kg/day, preferably 15 - 20 mg/kg/day or
the oral daily administrations of norucholic acid at a dose between 500 mg and 2000 mg and ursodeoxycholic acid at a dose of 13 - 23 mg/kg/day, preferably 15 - 20 mg/kg/day are between 6 - 10 hours, preferably 8 hours, apart from each other.

Thus in one embodiment, the present invention is directed to norucholic acid (NCA) for use in combination with an antifibrotic agent in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the antifibrotic agent selected from the group consisting of pirfenidone, fibrates, FXR-agonists, and combinations thereof,
wherein the norucholic acid is administered orally to the patient at a daily dose between 500 mg and 2000 mg.

In a further embodiment, the present invention is directed to norucholic acid (NCA) for use in combination with an antifibrotic agent in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the antifibrotic agent selected from the group consisting of pirfenidone, fibrates, FXR-agonists, and combinations thereof,
wherein the norucholic acid is administered orally to the patient at a daily dose between 500 mg and 2000 mg.

In a further embodiment, the present invention is directed to norucholic acid (NCA) for use in combination with an antifibrotic agent in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and

wherein the antifibrotic agent selected from the group consisting of pirfenidone, fibrates, FXR-agonists, and combinations thereof,
wherein the norucholic acid is administered orally to the patient at a daily dose between 500 mg and 2000 mg.

In a further embodiment, the present invention is directed to norucholic acid (NCA) for use in combination with an antifibrotic agent in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,

wherein the antifibrotic agent selected from the group consisting of pirfenidone, fibrates, FXR-agonists, and combinations thereof,
wherein the norucholic acid is administered orally to the patient at a daily dose between 500 mg and 2000 mg.

In a further embodiment, the present invention is directed to norucholic acid (NCA) for use in combination with an antifibrotic agent in the treatment of primary sclerosing cholangitis (PSC) in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the antifibrotic agent selected from the group consisting of pirfenidone, fibrates, FXR-agonists, and combinations thereof
wherein the norucholic acid is administered orally to the patient at a daily dose between 500 mg and 2000 mg for at least 12 weeks, preferably for at least 96 weeks.

In a further embodiment, the present invention is directed to norucholic acid (NCA) for use in combination with an antifibrotic agent in the treatment of primary sclerosing cholangitis (PSC) in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the antifibrotic agent selected from the group consisting of pirfenidone, fibrates, FXR-agonists, and combinations thereof
wherein the norucholic acid is administered orally to the patient at a daily dose between 500 mg and 2000 mg for at least 12 weeks, preferably for at least 96 weeks.

In a preferred embodiment, the antifibrotic agent is a fibrate selected from luminium clofibrate, bezafibrate, ciprofibrate, choline fenofibrate, clinofibrate, clofibrate, clofibride, fenofibrate, gemfibrozil, ronifibrate, simfibrate and combinations thereof.

In a preferred embodiment, the fibrate(s) are administered once daily and/or the daily dosage of the administered fibrates(s) is between 1 mg and 500 mg. Preferably, the daily dose of fibrate(s) is between 50 mg and 200 mg, more preferably between 80 mg and 120 mg. Preferably, the daily dose of fibrates is selected from 80 mg, 90 mg, 100 mg, 110 mg and 120 mg. Said amounts are administered preferably at once or possibly in more than one dose (at least 2, 3, 4, 5 or 10 doses) per day. The drug or the pharmaceutical composition may be administered for at least at least 12 weeks, more preferably at least 16 weeks, more preferably at least 20 weeks, more preferably at least 24 weeks, more preferably at least 30 weeks, more preferably at least 36 weeks, more preferably at least 44 weeks, more preferably at least 52 weeks, more preferably at least 60 weeks, more preferably at least 72 weeks, more preferably at least 84 weeks, and most preferably at least 96 weeks, in particular until resolution of PSC.

Another aspect of the present invention is directed to the norucholic acid for use in the treatment of a disease or a condition caused by and/or associated with PSC.

Thus in one embodiment, the present invention is directed to norucholic acid (NCA) for use in the treatment of a disease or a condition caused by and/or associated with PSC in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
wherein the norucholic acid is administered orally to the patient at a daily dose between 500 mg and 2000 mg.

In one embodiment, the present invention is directed to norucholic acid (NCA) for use in the treatment of a disease or a condition caused by and/or associated with PSC in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the disease or a condition caused by and/or associated with PSC is selected from Ulcerative colitis and Crohn's disease,
wherein the norucholic acid is administered orally to the patient at a daily dose between 500 mg and 2000 mg.

In a further embodiment, the present invention is directed to norucholic acid (NCA) for use in the treatment of a disease or a condition caused by and/or associated with PSC in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the disease or a condition caused by and/or associated with PSC is selected from Ulcerative colitis and Crohn's disease,
wherein the norucholic acid is administered orally to the patient at a daily dose between 500 mg and 2000 mg.

In a further embodiment, the present invention is directed to norucholic acid (NCA) for use in the treatment of a disease or a condition caused by and/or associated with PSC in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and
c) the patient has an Enhanced Liver Fibrosis (ELF) score ≥ 10.0, and

wherein the disease or a condition caused by and/or associated with PSC is selected from Ulcerative colitis and Crohn's disease,
wherein the norucholic acid is administered orally to the patient at a daily dose between 500 mg and 2000 mg.

In a further embodiment, the present invention is directed to norucholic acid (NCA) for use in the treatment of a disease or a condition caused by and/or associated with PSC in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and
c) the patient has an Enhanced Liver Fibrosis (ELF) score between 10.0 and 12.0,

wherein the disease or a condition caused by and/or associated with PSC is selected from Ulcerative colitis and Crohn's disease,
wherein the norucholic acid is administered orally to the patient at a daily dose between 500 mg and 2000 mg.

In a further embodiment, the present invention is directed to norucholic acid (NCA) for use in the treatment of a disease or a condition caused by and/or associated with PSC in a patient which is a human, wherein
a) the patient has a blood level of serum alkaline phosphatase (ALP) of ≤ 4.0 times upper limit of normal (ULN),
b) the patient has a liver stiffness of ≤ 15.0 kPa, preferably ≤ 12.5 kPa as measured by an elastometry technique such as vibration-controlled transient elastography (VCTE), and

wherein the disease or a condition caused by and/or associated with PSC is selected from Ulcerative colitis and Crohn's disease,
wherein the norucholic acid is administered orally to the patient at a daily dose between 500 mg and 2000 mg for at least 12 weeks, preferably for at least 96 weeks.

In a further embodiment, the present invention is directed to norucholic acid (NCA) for use in the treatment of a disease or a condition caused by and/or associated with PSC in a patient which is a human, wherein
a) the patient has a blood level of serum alkaline phosphatase (ALP) between 1.5 and 4.0 or between 1.5 and 3.5 or between 1.5 and 3.0 or between 1.5 and 2.5 times upper limit of normal (ULN),
b) the patient has a liver stiffness between 6.0 kPa and 15.0 kPa, preferably between 6.0 kPa and 12.5 kPa (6.0 kPa ≤ liver stiffness ≤ 15.0, preferably ≤ 12.5 kPa) as measured by an elastometry technique such as vibration-controlled transient elastography (VCTE), and

wherein the disease or a condition caused by and/or associated with PSC is selected from Ulcerative colitis and Crohn's disease,
wherein the norucholic acid is administered orally to the patient at a daily dose between 500 mg and 2000 mg for at least 12 weeks, preferably for at least 96 weeks.

### Description of the Figures

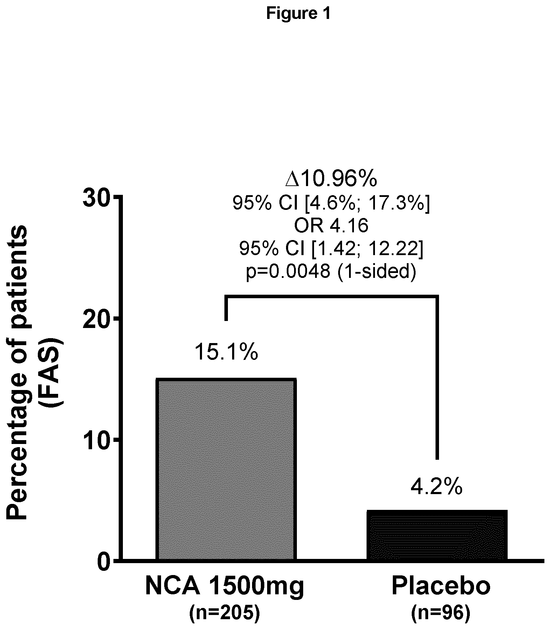

- **Figure 1**: shows a bar graph showing the percentage of PSC patients (Full Analysis Set [FAS] population) achieving a defined clinical response (partial normalization of ALP to <1.5× ULN and no worsening of Ludwig stage) at week 96 for NCA 1500 mg (n=205) versus placebo (n=96). NCA treatment resulted in a significantly higher response rate (15.1%) compared to placebo (4.2%), with a treatment difference of 10.96% (95% CI: 4.6%-17.3%), odds ratio of 4.16 (95% CI: 1.42-12.22), and p=0.0048 (1-sided). These findings provide strong evidence of the clinical efficacy of NCA in managing PSC.

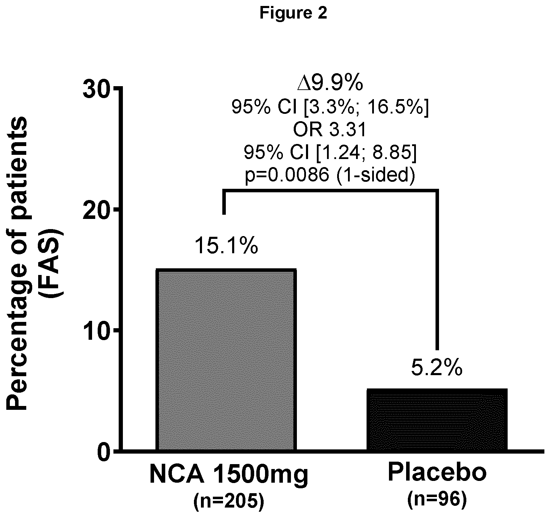

- **Figure 2**: shows a bar graph comparing the percentage of PSC patients (FAS population) achieving a defined clinical response (partial normalization of ALP <1.5 × ULN and no worsening of modified Nakanuma stage) at week 96 between NCA 1500 mg (n=205) and placebo (n=96). A significantly higher proportion of patients responded to NCA (15.1%) compared to placebo (5.2%), with a treatment difference of 9.9% (95% CI: 3.3%-16.5%), odds ratio of 3.31 (95% CI: 1.24-8.85), and p=0.0086 (1-sided). The superiority of NCA could be confirmed using a second histology score (mod. Nakanuma) with hierarchical testing.

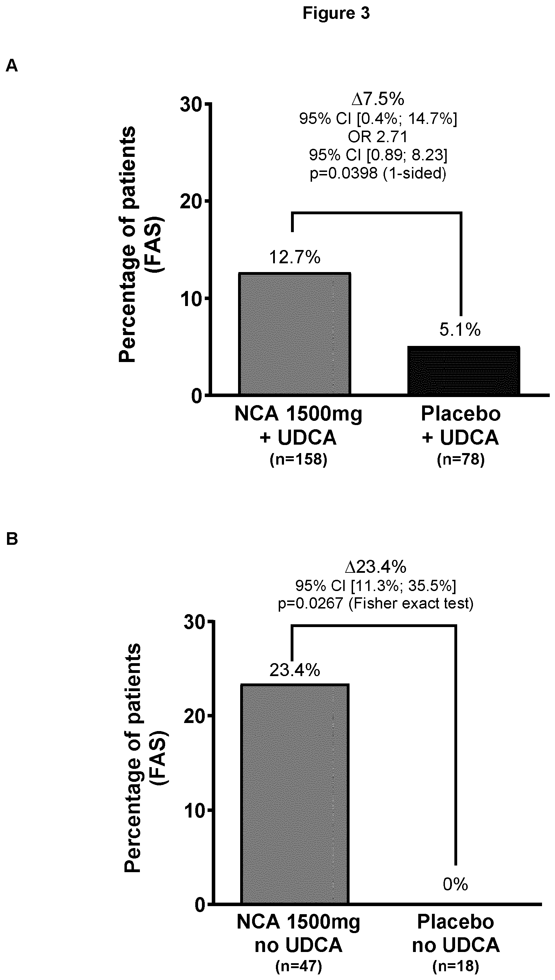

- **Figure 3**: (**A**) shows a bar graph showing the proportion of patients with clinical benefit at week 96 among those receiving NCA 1500 mg + UDCA (n=158) versus placebo + UDCA (n=78). NCA-treated patients had a higher response rate (12.7% vs. 5.1%), yielding a treatment difference of 7.5% (95% CI: 0.4%-14.7%; OR 2.71; p=0.0398, one-sided), demonstrating a benefit of NCA when co-administered with UDCA; (**B**) bar graph comparing the percentage of patients (FAS population) showing a defined clinical benefit at week 96 between NCA 1500 mg without UDCA (n=47) and placebo without UDCA (n=18). A significantly higher response rate was observed with NCA (23.4% vs. 0%), with a difference of 23.4% (95% Cl: 11.3%-35.5%; p=0.0267, Fisher exact test), supporting NCA's efficacy even in the absence of UDCA. The data demonstrate that NCA 1500 mg significantly improves clinical outcomes in PSC, both with and without coadministration of UDCA.

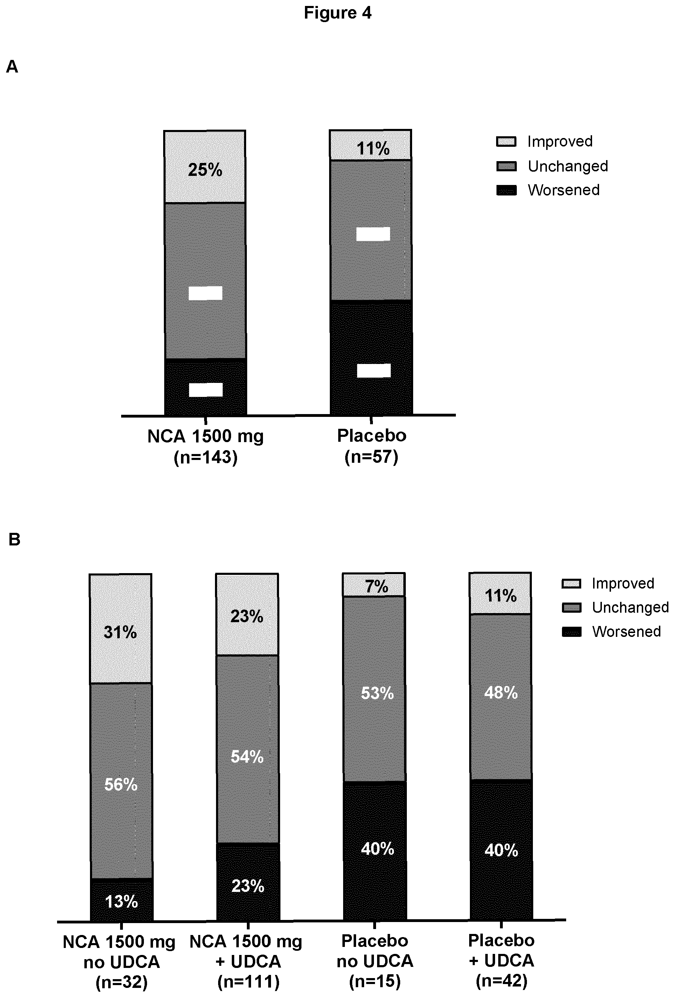

- **Figure 4**: (**A**) shows a stacked bar graph comparing overall response to treatment (Improved (>= 1 Ludwig stage), Unchanged, Worsened (>= 1 Ludwig stage)) at week 96 between NCA 1500 mg (n=143) and placebo (n=57). A greater proportion of NCA-treated patients improved (25% vs. 11%) and fewer worsened (20% vs. 40%) compared to placebo, supporting the efficacy of NCA in stabilizing or improving disease status in PSC; (**B**) stacked bar graph showing changes in patient status (Improved (>= 1 Ludwig stage), Unchanged, Worsened(>= 1 Ludwig stage)) by week 96, stratified by treatment group (NCA 1500 mg vs. placebo) and UDCA co-treatment. In both NCA subgroups (with and without UDCA), higher proportions of patients improved or remained unchanged compared to placebo. The rate of worsening was notably lower in the NCA groups, particularly without UDCA (13% vs. 40% in placebo groups), confirming that NCA reduces clinical deterioration in PSC. These data indicate more improvement and less worsening of histological disease stages by NCA treatment in comparison with placebo
- **Figure 5**: (**A**) shows a line graph representing mean alkaline phosphatase (ALP) levels over time. ALP remained consistently lower in the NCA group compared to placebo, which showed an increase by week 96; (**B**) Line graph showing mean gamma-glutamyl transferase (GGT) levels from baseline to week 96. Patients treated with NCA 1500 mg maintained lower and more stable GGT levels compared to the placebo group, which exhibited sustained elevation; (**C**) Line graph depicting mean ALT (alanine aminotransferase) levels over 96 weeks in PSC patients receiving NCA 1500 mg or placebo. ALT levels remained stable in the NCA group, while the placebo group showed an overall upward trend. These results demonstrate that NCA induces sustained reduction of liver enzymes which are biochemical markers associated with PSC progression. All values are mean (SEM).

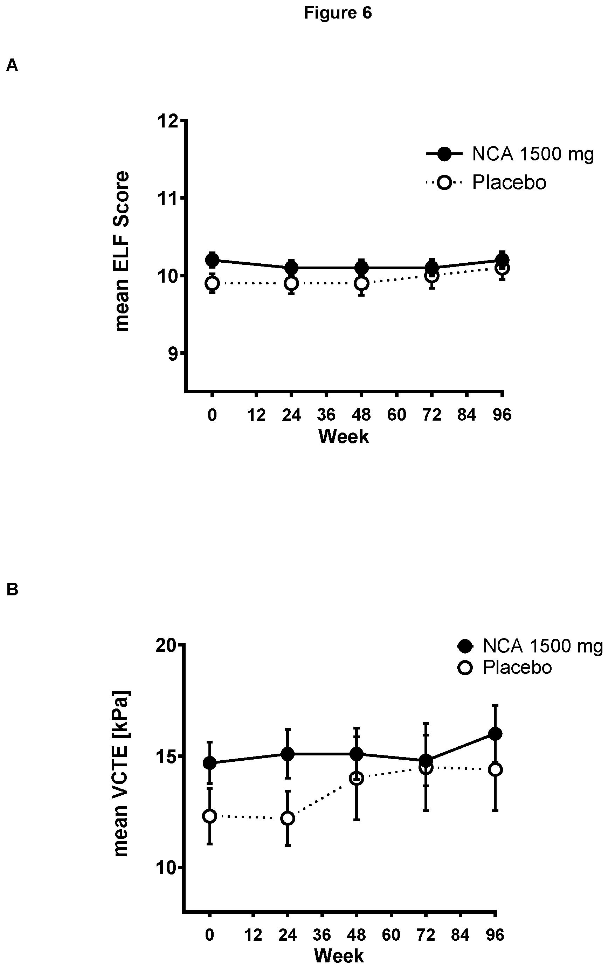

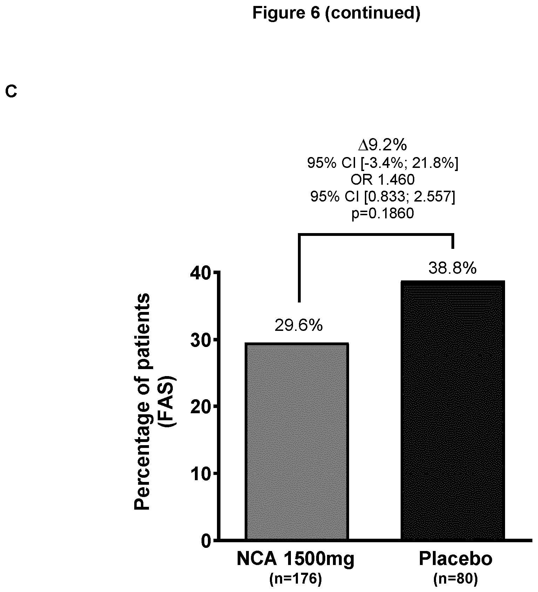

- **Figure 6**: (**A**) shows Line graph showing mean ELF (Enhanced Liver Fibrosis) scores over 96 weeks in patients receiving NCA 1500 mg or placebo. ELF scores remained stable in both groups, with NCA-treated patients exhibiting slightly higher mean scores than placebo at all timepoints; (**B**) Line graph showing mean liver stiffness over time, measured by VCTE (kPa), in PSC patients treated with NCA 1500 mg versus placebo. Measurements were taken at baseline and every 12 weeks up to week 96. Placebo group showed a gradual increase, whereas NCA-treated patients maintaining higher mean stiffness values throughout the study; (**C**) Bar graph comparing the percentage of patients (FAS population) with a specified outcome in the NCA 1500 mg group (n=176) versus placebo (n=80). The NCA group showed 29.6% versus 38.8% in placebo, a difference of -9.2% (95% CI: -3.4%, 21.8%), with an odds ratio of 1.460 (95% Cl: 0.833, 2.557), p=0.1860. Non-invasive fibrosis tests support histological analysis. Values are mean (SEM).

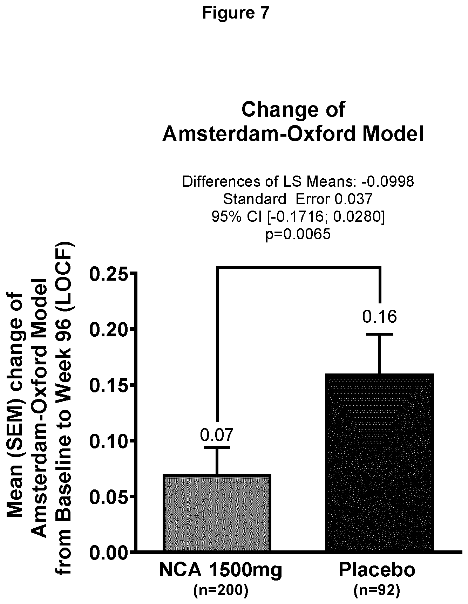

- **Figure 7**: shows a bar graph of the mean (±SEM) change in Amsterdam-Oxford Model scores from baseline to week 96 in PSC patients treated with NCA 1500 mg (n=200) versus placebo (n=92). Patients receiving NCA showed a smaller increase (0.07) compared to placebo (0.16), indicating reduced disease progression. The between-group difference was statistically significant (p=0.0065), with a least squares mean difference of -0.0998 (95% CI: -0.1716 to -0.0280).

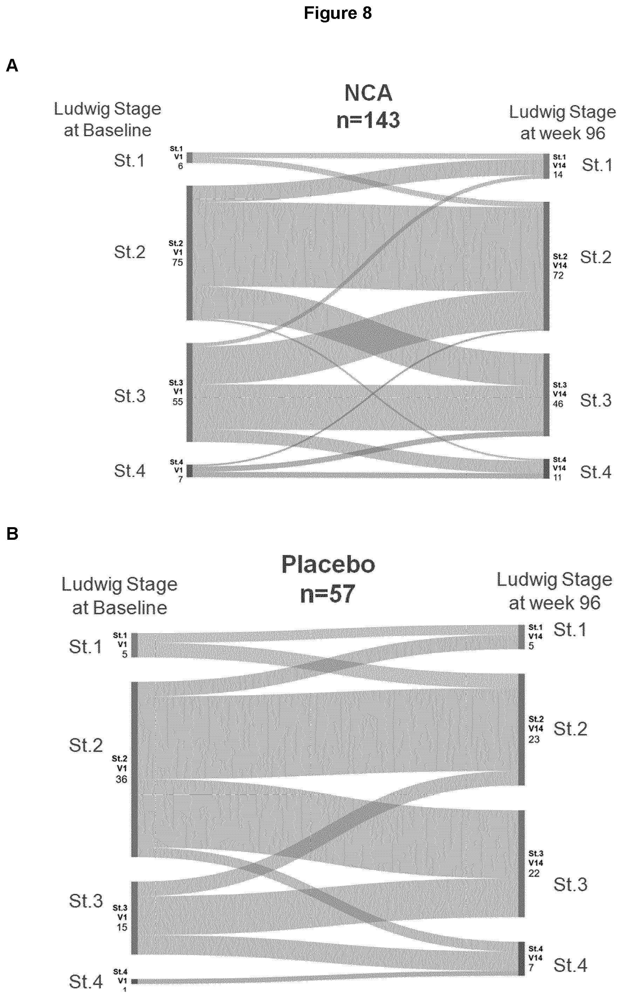

- **Figure 8**: (**A**) shows a Sankey diagram showing changes in Ludwig stage in 143 PSC patients treated with NCA over 96 weeks. Stages at baseline (left) and week 96 (right) are connected by flows representing the number of patients transitioning between stages. Most patients remained stable or improved, with fewer progressing to higher stages, indicating that NCA may help stabilize or reduce fibrosis in PSC; (**B**) Sankey diagram depicting changes in Ludwig stage over 96 weeks in 57 PSC patients receiving placebo. Ludwig stages at baseline (left) and week 96 (right) are connected by bands representing patient transitions. Most patients remained in the same stage or progressed, with limited evidence of regression, highlighting disease progression in the absence of active treatment.

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

A randomized, double-blind, placebo-controlled, parallel-group, multicenter Phase 3 clinical trial was conducted, designed to evaluate the efficacy and safety of NCA in adult patients with confirmed large-duct primary sclerosing cholangitis (PSC). A total of 301 subjects were randomized in a 2:1 ratio to receive either NCA 1500 mg orally once daily or a matching placebo once daily. Subjects were treated for a 96-week double-blind period, followed by an optional open-label extension period up to Week 192.

Eligible patients were aged 16 to 75 years with a confirmed diagnosis of PSC, serum alkaline phosphatase (s-ALP) greater than 1.5 times the upper limit of normal (ULN), and total bilirubin less than or equal to 4 mg/dL. Both UDCA-treated and UDCA-naïve patients were permitted, provided UDCA dosing was stable (not exceeding 20 mg/kg/day) for at least three months prior to screening.

### Inclusion Criteria:

Patients enrolled in the trial were required to meet the following criteria:
- Signed informed consent.
- Age ≥ 16 and ≤ 75 years, unless local regulations restricted enrollment of underaged participants.
- A confirmed diagnosis of PSC based on imaging modalities (retrograde, operative, percutaneous, or magnetic resonance cholangiography) within one year prior to baseline, showing characteristic intrahepatic and/or extrahepatic bile duct abnormalities.
- Availability of a liver biopsy obtained within two months prior to baseline.
- For patients pre-treated with ursodeoxycholic acid (UDCA), confirmation of a stable UDCA dose for at least three months prior to baseline not exceeding 20 mg/kg/day.
- Screening serum alkaline phosphatase > 1.5 times ULN.
- Patients with or without inflammatory bowel disease (IBD) were eligible. For those without definitive exclusion of IBD, a colonoscopy with segmental biopsy prior to baseline was required. For those with IBD, a colonoscopy within 12 months prior to baseline was necessary, with additional requirements:
   - Patients with Crohn's disease (CD) had to be in remission with a Crohn's Disease Activity Index (CDAI) < 150.
   - Patients with ulcerative colitis (UC) had to be in remission or exhibit mild disease with a Clinical Activity Index (CAI) < 8.
- Female participants of childbearing potential had to use highly effective contraceptive methods throughout the treatment period and for at least four weeks following the last dose. Acceptable contraception included hormonal methods that inhibit ovulation, intrauterine devices, intrauterine hormone-releasing systems, bilateral tubal occlusion, vasectomized partners, or consistent sexual abstinence. Additional use of male condoms was recommended for non-injectable hormonal methods. Women not of childbearing potential due to surgical sterilization or postmenopause (≥ 2 years) were also eligible.

### Exclusion Criteria:

Patients who met any of the following criteria were excluded from the study:
- History or presence of other concomitant liver diseases, including:
   - Positive Hepatitis B or C serology (HBsAg+, anti-HBc+, anti-HCV+). Note: anti-HBc+ patients may be included if HBV-DNA is negative.
   - Primary Biliary Cholangitis (positive AMA titer).
   - Wilson's Disease.
   - Hemochromatosis.
   - Autoimmune Hepatitis.
   - Chronic alcoholic consumption (> 30 g/day).
   - Biopsy-proven Non-alcoholic Steatohepatitis (NASH).
   - Cholangiocarcinoma.
- Treatment with any of the following drugs within the last 3 months prior to baseline: any glucocorticosteroids (including budesonide), if not inhalational, azathioprine or other immunosuppressive drugs (e.g., cyclophosphamide, cyclosporine, methotrexate, tacrolimus, 6-mercaptopurine), chlorambucil, pentoxifylline, penicillamine, pirfenidone, biologics (e.g., anti-tumor necrosis factor-α therapy, integrin-antagonists (vedolizumab)), or rifampicin.
- Dose changes within the last 6 months of concomitant treatment with vitamin D or fibrates.
- Secondary causes of sclerosing cholangitis, including iatrogenic bile duct injury, IgG4-associated cholangitis (IgG4 > 4× ULN), sepsis, or burns.
- Small duct cholangitis without large duct disease.
- Liver cirrhosis with Child-Pugh score ≥ 9, or history/presence of hepatic decompensation (e.g., variceal bleeding).
- Hepatic encephalopathy or poorly controlled ascites.
- Endoscopic treatment for bile duct stenosis planned or required within 5 months post-randomization.
- Cholangiosepsis requiring hospitalization within 3 months prior to baseline.
- Current episode of suppurative cholangitis.
- Total bilirubin > 4.0 mg/dL at screening or baseline.
- IBD requiring specific treatment within 3 months prior to baseline, excluding 5-ASA compounds or probiotics.
- Any known relevant infectious disease (e.g., active TB, AIDS-defining conditions).
- Abnormal renal function: GFR (cystatin C) < 30 mL/min.
- TSH > ULN at screening (acceptable if fT4 is normal).
- Any severe cardiovascular, renal, endocrine, or psychiatric disorder that may interfere with compliance or interpretation of results, including atrial fibrillation.
- Any active malignancy.
- Known hypersensitivity to NCA or structurally related compounds.
- Pregnancy or breastfeeding.
- Participation in another clinical trial within 30 days prior to screening, or previous participation in this study.
- Absolute contraindication for liver biopsy.
- Patients who are imprisoned, institutionalized, under legal guardianship, or otherwise unable to provide informed consent.

### Endpoints and Results:

The primary endpoint was a composite assessed at the end of the double-blind treatment period (Week 96 or earlier if the patient withdrew). Fulfillment of this composite primary endpoint required the patient to achieve both normalization or partial normalization of serum alkaline phosphatase to less than 1.5 times the ULN, and no histological worsening of liver fibrosis as determined by the Ludwig histological staging system.

The key secondary endpoint at the end of the double-blind treatment period (Week 96 or earlier if the patient withdrew) was a composite assessed at the end of the double-blind treatment period (Week 96 or earlier if the patient withdrew). Fulfillment of this composite primary endpoint required the patient to achieve both partial normalization of serum alkaline phosphatase to less than 1.5 times the ULN, and no histological worsening of liver fibrosis as determined by modified Nakanuma staging. The modified Nakanuma staging included the substitution of Orcein staining by immunohistochemical staining of cytokeratin 7 (CK7) to assess chronic cholestasis score.

Further secondary endpoints included Liver stiffness measurements (Fibroscan), ELF score and Amsterdam Oxford Prognosis score.

Liver biopsies were collected at baseline (between Week -8 and Week -4), at Week 96, and optionally at Week 192. Secondary endpoints included changes in liver enzymes (including ALT, AST, and GGT), bilirubin levels, liver stiffness, histologic improvement defined as a one-stage reduction in Ludwig score, proportion of patients with full biochemical remission, and incidence of clinical events such as cirrhosis complications, malignancies, liver transplantation, or death.

The results demonstrated that a significantly higher proportion of NCA-treated patients achieved the composite primary endpoint compared to placebo. NCA treatment resulted in sustained reductions in s-ALP and improvements in ALT, AST, and GGT over the 96-week period. Histological improvement, defined as a reduction in Ludwig fibrosis stage by at least one stage, occurred in 25.2% of NCA-treated patients compared to 10.5% in the placebo group (p = 0.0217). In contrast, the proportion of patients with fibrosis worsening of one or more Ludwig stages was significantly lower in the NCA group (20.3%) compared to placebo (40.4%) (p = 0.0069).

Treatment with NCA was well tolerated, with no significant differences in pruritus, fatigue, or quality of life measures between the treatment arms as assessed by CLDQ and CLDQ-PSC instruments. The overall incidence of clinical events was similar between treatment groups, despite a higher proportion of NCA-treated patients presenting with advanced fibrosis (Ludwig stage 3 or 4) at baseline. This finding suggests a potential protective effect of NCA in high-risk patients.

The safety profile of NCA was comparable to placebo. Treatment-emergent adverse events (TEAEs) occurred in 97.6% of NCA patients and 92.7% of placebo patients. The most common TEAEs included pruritus (34.0% NCA vs. 30.2% placebo), nasopharyngitis, diarrhea, and headache. Serious TEAEs occurred in 39.3% of NCA patients and 35.4% of placebo patients, with study-drug-related serious AEs being low (1.9% vs. 2.1%). One death (0.5%) occurred in the NCA group.

**Table 1: Safety Profile Summary**

| **TEAEs, n (%)** | **NCA 1500 mg (N = 206)** | **Placebo (N = 96)** |
|---|---|---|
| Any TEAE | 201 (97.6%) | 89 (92.7%) |
| Diarrhea | 33 (16.0%) | 16 (16.7%) |
| SARS-CoV-2 infection | 36 (17.5%) | 12 (12.5%) |
| Nasopharyngitis | 42 (20.4%) | 16 (16.7%) |
| Headache | 33 (16.0%) | 18 (18.8%) |
| Pruritus | 70 (34.0%) | 29 (30.2%) |
| TEAE related to study drug | 97 (47.1%) | 40 (41.7%) |
| TEAE leading to discontinuation | 13 (6.3%) | 10 (10.4%) |
| TE serious AE | 81 (39.3%) | 34 (35.4%) |
| TE serious AE related to study drug | 4 (1.9%) | 2 (2.1%) |
| Death | 1 (0.5%) | - |

Baseline fibrosis and stiffness were measured using non-invasive tests such as vibration-controlled transient elastography (VCTE) and Enhanced Liver Fibrosis (ELF) score. Median VCTE values were 10.4 kPa for NCA and 8.5 kPa for placebo. Most NCA patients (55.6%) had severe fibrosis (ELF ≥ 9.8), compared to 49.5% in the placebo group.

**Table 2: Baseline Characteristics Summary**

| **Characteristic** | | **NCA 1500 mg (N = 205)** | **Placebo (N = 96)** |
|---|---|---|---|
| **VCTE (median) [kPa]** | | 10.4 (7.3; 17.5)n=188 | 8.5 (6.5; 11.9)n=88 |
| | Stage 0: <7.4 | 48 (25.5%) | 28 (31.8%) |
| | Stage 1: 7.4-8.5 | 16 (8.5%) | 17 (19.3%) |
| | Stage 2: 8.6-9.5 | 15 (8.0%) | 6 (6.8%) |
| | Stage 3: 9.6-14.3 | 47 (25.0%) | 24 (27.3%) |
| | Stage 4: ≥14.4 | 32 (33.0%) | 13 (14.8%) |
| **ELF Score (median)** | | 9.9 (9.2, 11.0) | 9.7 (9.1, 10.4) |
| | No to mild fibrosis (<7.7) | 1 (0.49%) | 2 (2.11%) |
| | Moderate fibrosis (7.7-<9.8) | 90 (43.90%) | 46 (48.42%) |
| | Severe fibrosis (≥9.8) | 114 (55.61%) | 47 (49.47%) |

### Conclusion:

NCA is superior to placebo in the primary efficacy variable (partial normalization of ALP combined with no worsening of Ludwig stage), a finding confirmed by a second independent histological score (modified Nakanuma staging). The response rate for NCA was higher than for placebo, both with and without concomitant UDCA use. More patients in the NCA arm experienced histological improvement and fewer experienced worsening of fibrosis compared to placebo. NCA induced a sustained reduction in liver enzymes. Non-invasive fibrosis assessments (VCTE and ELF) and the Amsterdam-Oxford score support the histological findings. NCA was well tolerated and had a safety profile comparable to placebo.

## Claims

1. Norucholic acid for use in the treatment of primary sclerosing cholangitis in a patient which is a human, wherein
a) the patient has a blood level of serum alkaline phosphatase of ≤ 4.0 times upper limit of normal, and
b) the patient has a liver stiffness of ≤ 15.0 kPa as measured by an elastometry technique, and
wherein the norucholic acid is administered orally to the patient at a daily dose between 500 mg and 2000 mg.

2. The norucholic acid for use according to claim 1, wherein the patient has a blood level of serum alkaline phosphatase between 1.5 times upper limit of normal and 4.0 times upper limit of normal.

3. The norucholic acid for use according to claim 1 or 2, wherein the patient has an Enhanced Liver Fibrosis score between 10.0 and 12.0.

4. The norucholic acid for use according to any one of claims 1 to 3, wherein the norucholic acid is administered orally daily to the patient for at least 96 weeks.

5. The norucholic acid for use according to any one of claims 1 to 4, wherein the patient has serum levels ≥ 1.5 times upper limit of normal of one or more of alanine aminotransferase or aspartate aminotransferase.

6. The norucholic acid for use according to any one of claims 1 to 5, wherein the patient suffers from large duct cholangitis.

7. The norucholic acid for use according to any one of claims 1 to 6, wherein the patient is not treated with one or more medications selected from: a glucocorticoid, an immunosuppressive drug, chlorambucil, pentoxifylline, penicillamine, pirfenidone, rifampicin, an integrin-antagonist or an anti-tumor necrosis factor-α for at least 3 months prior to the start of norucholic acid treatment.

8. The norucholic acid for use according to any one of claims 1 to 7, wherein the patient exhibits positive anti-nuclear antibody and/or perinuclear anti-neutrophil cytoplasmic antibody serology, and negative antimitochondrial antibody serology.

9. The norucholic acid for use according to any one of claims 1 to 8 in the treatment of a disease or a condition caused by and/or associated with PSC selected from Ulcerative colitis and Crohn's disease.

10. The norucholic acid for use according to any one of claims 1 to 9, wherein the patient is concurrently administered ursodeoxycholic acid orally daily at a dose of 13 - 23 mg/kg/day, preferably 15 - 20 mg/kg/day.

11. The norucholic acid for use according to claim 10, wherein norucholic acid is administered daily once in the morning and ursodeoxycholic acid is administered daily once in the evening or wherein the administrations of norucholic acid and ursodeoxycholic acid are between 6 - 10 hours, preferably 8 hours, apart from each other.

12. The norucholic acid for use according to any one of claims 1 to 11, in combination with an antifibrotic agent selected from the group consisting of pirfenidone, fibrates, FXR-agonists, and combinations thereof.

13. The norucholic acid for use according to claim 12, wherein the antifibrotic agent is a fibrate selected from luminium clofibrate, bezafibrate, ciprofibrate, choline fenofibrate, clinofibrate, clofibrate, clofibride, fenofibrate, gemfibrozil, ronifibrate, simfibrate and combinations thereof.

14. The norucholic acid for use according to any one of claims 1 to 13, wherein the elastometry technique is vibration-controlled transient elastography.
